# EUROPEAN PATENT SPECIFICATION

(11) **EP 2 382 319 B1**
(45) Date of publication and mention of the grant of the patent: **23.03.2016**
(21) Application number: 10701462.3
(22) Date of filing: 19.01.2010
(51) Int. Cl.: C12N 15/68, C12N 9/22, C12P 21/02, C12N 15/85

(54) **A METHOD FOR INCREASING PROTEIN EXPRESSION IN CELLS**
METHODE ZUR ERHÖHUNG DER PROTEINEXPRESSION IN ZELLEN
MÉTHODE POUR AUGMENTER L'EXPRESSION DES PROTEINES DANS DES CELLULES

(30) Priority: 19.01.2009 WO PCT/EP2009/000302
(43) Date of publication of application: 02.11.2011
(73) Proprietor: King Faisal Specialist Hospital And Research Centre, Riyadh 11211 (SA)
(72) Inventor: KHABAR, Khalid S. Abu, Riyadh 11211 (SA)
(74) Representative: Engelhard, Markus
(86) International application number: PCT/EP2010/000271
(87) International publication number: WO 2010/081741

(56) References cited:
- LI X-L ET AL: "RNase-L-dependent destabilization of interferon-induced mRNAs: A role for the 2-5A system in attenuation of the interferon response" JOURNAL OF BIOLOGICAL CHEMISTRY, AMERICAN SOCIETY OF BIOLOCHEMICAL BIOLOGISTS, BIRMINGHAM, US, vol. 275, no. 12, 24 March 2000 (2000-03-24), pages 8880-8888, XP002153781 ISSN: 0021-9258 cited in the application
- HAN JIAN-QIU ET AL: "Sensitivity of hepatitis C virus RNA to the antiviral enzyme ribonuclease L is determined by a subset of efficient cleavage sites" JOURNAL OF INTERFERON AND CYTOKINE RESEARCH, vol. 24, no. 11, November 2004 (2004-11), pages 664-676, XP002525826 ISSN: 1079-9907 cited in the application
- KHABAR KHALID S A ET AL: "RNase L mediates transient control of the interferon response through modulation of the double-stranded RNA-dependent protein kinase PKR." JOURNAL OF BIOLOGICAL CHEMISTRY, vol. 278, no. 22, 30 May 2003 (2003-05-30) , pages 20124-20132, XP002525827 ISSN: 0021-9258 cited in the application
- WRESCHNER D H ET AL: "INTERFERON ACTION-SEQUENCE SPECIFICITY OF THE PPP(A2'P)NA-DEPENDENTRIBONUCLEASE" NATURE, NATURE PUBLISHING GROUP, LONDON, UK, vol. 289, 29 January 1981 (1981-01-29), pages 414-417, XP000941867 ISSN: 0028-0836
- CHANDRASEKARAN ET AL: "RNase-L regulates the stability of mitochondrial DNA-encoded mRNAs in mouse embryo fibroblasts" BIOCHEMICAL AND BIOPHYSICAL RESEARCH COMMUNICATIONS, ACADEMIC PRESS INC. ORLANDO, FL, US, vol. 325, no. 1, 3 December 2004 (2004-12-03), pages 18-23, XP005027195 ISSN: 0006-291X cited in the application
- KHABAR ET AL: "Post-transcriptional control of the interferon system" BIOCHIMIE, MASSON, PARIS, FR LNKD- DOI:10.1016/J.BIOCHI.2007.02.008, vol. 89, no. 6-7, 1 June 2007 (2007-06-01) , pages 761-769, XP022138017 ISSN: 0300-9084

## Description

The present invention relates to a method for increasing the expression of a protein in cells, preferably in eukaryotic cells by reducing the number of RNase L cleavage sites in the coding and/or non-coding region of the nucleic acid sequence of said protein. Furthermore, it relates to nucleic acid sequences exhibiting a reduced number of RNase L cleavage sites as well as to the proteins translated from such sequences.

Transient response genes regulate critical biological responses that include cell proliferation, signal transduction events, and responses to exogenous agents, such as inflammatory stimuli, microbes, and radiation (Lai et al., 2006; Lal et al., 2004; Lopez de Silanes et al., 2005). They are controlled by both cis-acting factors such as specific sequence elements and trans-acting factors like certain RNA binding proteins. Sequence elements, mainly in the 3' untranslated region (3'UTR) and modulation by RNA binding proteins can affect messenger RNA (mRNA) stability and protein translation.

An appreciable number of genes harbor destabilizing sequence elements in the 3' UTR of their mRNA, mostly adenylate-uridylate (AU)-rich elements (AREs). These AREs comprise a heterogeneous group of sequence classes that can affect protein interactions with the mRNA, and therefore influence the mRNA decay characteristics (Bakheet et al., 2006; Barreau et al., 2005). The stabilization of cellular mRNAs can occur by the activity of mRNA stabilization-promoting proteins, such as the HuR protein, or by inactivation of RNA decay promoting proteins, such as the zinc finger protein tristetraprolin (TTP).

A different class of trans-acting factors that can affect cellular mRNAs is the endoribonuclease Ribonuclease L (RNase L), which is an ubiquitous intracellular enzyme that has previously been thought to be specific to viral mRNAs. However, recent studies showed that RNase L can also participate in the transient response of certain biological processes (Khabar et al., 2003a; Li et al., 2000). RNase L is considered to be a part of the interferon (IFN) system. IFN induces gene expression of the enzyme oligoadenylate synthetase (OAS) which, upon binding to viral double-stranded RNA intermediates, becomes activated and synthesizes short 2'-5'oligoadenylates (2-5A). These, in turn, activate RNase L, which potently degrades viral mRNAs. RNase L is activated by subnanomolar levels of 2-5A, resulting in the cleavage of single-stranded regions of viral RNA, preferentially after UU and UA dinucleotides in viral mRNAs (Wrechester et al., 1981; Han et al., 2004). At higher levels, RNase L may lead to broader effects such as cleavage of 18 S and 28 S ribosomal RNAs (Wreschner et al., 1981).

In recent years it has become widely accepted that RNase L participates in the degradation of selected cellular mRNAs (Bisbal et al., 2000; Chandrasekaran et al., 2004; Khabar et al., 2003b; Le Roy et al., 2001; Li et al., 2000). Specifically, RNase L has been shown to down-regulate PKR mRNA (Khabar et al., 2003b). During the IFN antiviral response in normal cells, PKR mRNA expression is transient, but in RNase L-null cells, extended kinetics of PKR mRNA expression is observed due to increased mRNA stability (Khabar et al., 2003b). The effect results in prolongation of the PKR-dependent phosphorylation of the subunit of eukaryotic translation initiation factor 2, eIF2α, a process that leads to inhibition of viral protein synthesis (Khabar et al., 2003b). Thus, RNase L contributes to the transient nature of the IFN response in order to ensure a brief translational arrest imparted by PKR. A similar role of RNase L negative regulation of the IFN response has also been suggested by the report that a novel IFN-stimulated gene encoding a 43-kDa ubiquitin-specific protease, designated ISG43, is down-regulated by RNase L (Li et al., 2000). This regulation occurs at the level of mRNA stability, since the ISG43 mRNA half life increases in RNase L-null cells (Li et al., 2000). RNase L can down-regulate another functionally important cellular mRNA, myoD, encoding an import transcription factor essential for muscle differentiation. RNase L and its inhibitor RLI are sequentially induced during C2 cell line myoblast differentiation to myotubes (Bisbal et al., 2000). Inhibition or over-expression of RNase L prolongs or decreases MyoD mRNA half life, respectively (Bisbal et al., 2000). Since a pool of RNase L molecules localizes to mitochondria and is increased following IFN-α treatment, a role of RNase L in down-regulating mitochondrial mRNAs, such as those of CYTB, ATPase 6 (ATP6), and cytochrome oxidase II (CO), has been proposed as a mechanism of the anti-proliferative action of IFN (Le Roy et al., 2001). This was demonstrated by reducing RNase L activity through the introduction of an antisense construct or by directly activating RNase L activity by 2-5A (Le Roy et al., 2001). It has further been shown that RNase L exhibits a preference for viral mRNA, for example encephalomyocarditits virus (EMCV), when compared to non-viral mRNAs, particularly in conditions where the levels of 2-5A is limiting. (Li et al., 1998a). The effects of RNase L on cellular mRNAs appear to be highly restricted to specific mRNAs, since no global effects on cellular mRNAs are observed in the studies that have dealt with this topic. Furthermore, none of the above studies demonstrates a direct binding of RNase L to cellular target mRNAs or a sequence specificity as shown for viral mRNAs. Thus, the mechanism of RNase L activity in connection with non-viral cellular mRNAs remains largely unclear.

Dominant negative forms of RNase L have previously been generated by either amino acid substitutions or by truncation of the full length protein. For example, a dominant negative RNase L, ZB1, inhibits the antiviral and anti-proliferative action of wild type RNase L; it is a truncated form of murine RNase L, which lacks 89 carboxy-terminal amino acids (Hassel et al., 1993). Dong et al., 2001 describe other truncations and point mutations of RNase L, e.g. mutations in the nuclease domain (R667A).

U.S. patent 6,762,038 suggests the use of mutant embryonic fibroblasts cell lines (MEFs) generated from mice having a homozygous disruption in their RNase L gene (Zhou et al., 1997) for enhanced expression of transfected genes. However, the effect of enhanced expression is restricted to these particular cell lines. Furthermore, the creation of new RNase L-null cell lines is a very laborious process and not readily applicable to all mammalian and/or eukaryotic expression systems.

Mammalian expression systems have become an important means for therapeutic protein and antibody production and possess several advantages over prokaryotic expression systems, e.g. with respect to proper protein folding and posttranslational modifications, such as glycolysation. However, in many systems, low protein expression yields represent a costly technical obstacle. This is particularly problematic, if the desired protein is inherently difficult to express, e.g. membrane proteins, such as G-protein linked receptors (GPCRs), large proteins, antibodies, fusion proteins, protein complexes, vaccines, and blood plasma proteins. Reasons for this difficulty could be an inherent instability of the protein itself, an inherent instability of the mRNA, as it is the case for AU-rich elements containing mRNAs, or weak promoter activity.

Common solutions to these problems focused on increasing the expression of proteins by providing strong promoters, such as the CMV promoter, and enhancer elements upstream or downstream of the promoter including specific types of introns, such as intron A of CMV. Other solutions involved chromatin Matrix Attachment Region (MAR) elements which are 300-3000 base pairs long DNA elements that are important in nuclear and chromosomal architecture. It was proposed that these elements prevent the neighbouring chromatin from affecting transgene expression, which leads to an increased probability of isolating a clone exhibiting the desired regulated expression (U.S. patent 7,129,062). This particular approach is potentially problematic as it may involve several different vector constructs to achieve the effect. Despite the availability of these approaches, there is still a need to further increase the protein expression of proteins that are difficult to express, such as those mentioned above.

Codon optimization (or codon usage optimization) is another method known in the art to boost protein production. It is based on the observation that, if a nucleic acid sequence encoding the protein to be expressed contains codons (triplets) that are rarely used by the host, its expression level will not be maximal. Codon optimization basically involves altering the rare codons in the target nucleic acid sequence, so that they more closely reflect the codon usage of the host. The information usually used for the optimization process is therefore the DNA or protein sequence to be optimized and a codon usage table of the respective host (see for example Table 1 for the human genome). The codon usage table lists the relative frequency of each possible codon for a particular amino acid in a given organism. A full list of codon usage bias in all organisms is found on the website: http://www.kazusa.or.jp/codon/. Several web-based programs are also available to optimize codons based on codon usage bias for a given host organism. Codon optimization may be successful in some situations where genes of non-human or non-mammalian origin are expressed in human or other mammalian host cells, and vice versa. However, codon usage is just one of many factors influencing the expression level of a protein, and the effect of codon optimization is often limited.

It was an object of the present invention to provide for a method to significantly improve the yield of endogenous and exogenous (recombinant) proteins expressed in cells of any organism, preferably in eukaryotic cells, including proteins that are inherently difficult to express. This method should be time and cost efficient and should allow for the large-scale production of proteins in cells of any type of organism including prokaryotic and eukaryotic cells. It was another object of the present invention to achieve this improvement in expression yields without changing regulatory approved features for the production of recombinant proteins, such as the cell lines used, recombinant protein characteristics, or the use of exogenous materials, in order to allow for the production of therapeutically used proteins.

The objects of the present invention are solved by a method for increasing the expression of a protein in eukaryotic cells, said method comprising the step of reducing the number of RNase L cleavage sites in the nucleic acid sequence of said protein.

The nucleic acid sequence of a protein comprises both coding and non-coding regions, i.e. regions that are translated into a sequence of amino acids (also referred to as exons) and regions that are not translated into a sequence of amino acids. Non-coding regions of the nucleic acid sequence are for example the 5' untranslated region (5'UTR), the 3' untranslated region (3'UTR), and introns. All of these elements (5'UTR, 3'UTR, introns, and the coding region) can control gene and protein expression, and are, thus, targets for the above method. According to the invention, said step of reducing the number of RNase L cleavage sites reduces said number either in the coding region or non-coding region, or in both.

In one embodiment said number of RNase L cleavage sites is reduced by at least 10%, preferably at least 25%, more preferably at least 50% (compared to the number of RNase L cleavage sites in the wild type nucleic acid sequence).

Preferably said cleavage sites are UU and/or UA dinucleotides.

RNase L is an endoribonuclease, and is, thus, only active on the RNA level (both primary RNA, i.e. unspliced, and mRNA, i.e. spliced). UU and UA dinucleotides only occur on the RNA level.
However, it is preferred that said step of reducing the number of RNase L cleavage sites in the nucleic acid sequence of said protein is performed on the DNA level: UU and UA dinucleotides in an RNA sequence correspond to TT and TA dinucleotides in a DNA sequence. Techniques that allow to specifically change a given DNA sequence are well know in the art and include, but are not limited to gene synthesis, site-directed mutagenesis, deletion mutations by restriction digestion, and mutation introduction by recombination. The technique of gene synthesis is particularly preferred according to the present invention.

In one embodiment of the present invention said step of reducing the number of RNase L cleavage sites reduces said number in the coding region of said nucleic acid sequence.

Preferably said step of reducing the number of RNase L cleavage sites in said nucleic acid sequence is performed without altering the amino acid sequence of said protein. The open reading frame (ORF) is, thus, not altered by said step.

In one embodiment in said step of reducing the number of RNase L cleavage sites a codon comprising a UU and/or UA dinucleotide is exchanged for an alternative codon not comprising a UU and/or UA dinucleotide and coding for the same amino acid.

In one embodiment in said step of reducing the number of RNase L cleavage sites at least one codon of an adjacent pair of codons comprising a UU and/or UA dinucleotide is exchanged for an alternative codon coding for the same amino acid so that said adjacent pair of codons does no longer comprise a UU and/or UA dinucleotide.

Preferably the first codon of said adjacent pair of codons comprising a UU and/or UA dinucleotide is exchanged.

In one embodiment said alternative codon is the more frequently used codon in said eukaryotic cells.

In one embodiment of the present invention said step of reducing the number of RNase L cleavage sites reduces said number in the non-coding region of said nucleic acid sequence.

Preferably said non-coding region is a 5'UTR, a 3'UTR, or an intron.

Examples for introns include, but are not limited to the CMV intron, SV40 intron, rabbit beta globin intron (RBTG), and synthetic introns.

In one embodiment said step of reducing the number of RNase L cleavage sites in the non-coding region of said nucleic acid sequence is performed by mutation, deletion, or insertion of nucleotides.

Preferably said step of reducing the number of RNase L cleavage sites in the non-coding region of said nucleic acid sequence does not alter functionally important elements in the non-coding region, such as sequences in the 5'UTR that are close to the initiation codon (ATG), since they may harbor translation enhancing sequences (e.g. kozac), the poly A signal in the 3'UTR (e.g. AAUAAA or AUUAAA) or other necessary or accessory sequence elements used for polyadenylation, intron-exon junctions/boundaries, splicing branch points and exon donor/acceptor splice sites in introns, and the CT-rich area between the splice acceptor site to the end of the branch point. For example there is an U-rich 50 nucleotide region that is downstream of the strong poly A signal, which should not be altered when possible (Legendre and Gautheret, 2003).

Preferably said step of reducing the number of RNase L cleavage sites in the non-coding region does not
(a) change the GC content of an intron to more than 80% and its length to less than 80%,
(b) change the GC content of a 5'UTR to more than 80%, and
(c) change the GC content of a 3'UTR to more than 80% and its length to less than 80%.

In one embodiment above method further comprises the step of codon optimization prior to said step of reducing the number of RNase L cleavage sites.

In one embodiment above method further comprises the step of transfecting said nucleic acid sequence of said protein into said eukaryotic cells in form of an expression active PCR product or contained in an expression vector after said step of reducing the number of RNase L cleavage sites.

The term "expression active PCR product" as used herein is meant to refer to a PCR product that is generated by PCR amplification using two primers complementary to sequences flanking the DNA sequence of interest, such as a cDNA, an open reading frame, or a gene that is contained in an expression vector, wherein the resulting PCR product contains a promoter, the DNA sequence of interest, and a termination sequence, and allows the expression of the DNA of interest, when transfected to a host cell (see also: Al-Zoghaibi et al., 2007).

According to the present invention any expression vector can be used, however, eukaryotic/mammalian expression vectors are preferable. Mammalian expression vectors are widespread tools to study the biological function of a protein, and various types (e.g. plasmid-based or viral-based vectors) are known in the art. Suitable expression vectors are not limited to a specific promoter, 5'UTR, 3'UTR, or intron. They can be constitutively expressed, inducible, repressed or regulatable.

According to the invention, it is preferred that the promoter is eukaryotic and the termination site is a poly A sequence containing a polyadenylation signal. Eukaryotic or mammalian promoters are known in the art and include, but are not limited to cytomegalovirus (CMV) immediate early promoter, SV40 promoter, elongation factor (EF) promoter, RSV promoter, and chicken β-actin promoter. Preferred eukaryotic polyadenylation signals are bovine growth factor (BGH) poly site, growth hormone poly A, SV40 poly A, and HSK poly A (Foecking and Hofstetter, 1986; Kobayashi et al., 1997). Examples of eukaryotic terminators are bovine growth factor (BGH) poly A site, SV40 poly A, HSK poly A, and synthetic poly A.

Methods for transiently or stably transfecting cells with DNA/vectors are well known in the art. These include, but are not limited to calcium phosphate co-precipitation, electroporation, cationic polymer transfection, and liposome-mediated transfection (e.g. lipofection). Reagents for liposome-mediated transfection are commercially available, e.g. lipofectamine (Invitrogen) and polyethylenimine (Sigma). Cells can also be transfected by viral transfection or via viral coat particles. Another preferred method for the transfection of cells according to the present invention is the *in vivo* microinjection of said expression active PCR product or said expression vector and selectively growing the cells containing said expression active PCR product or said expression vector with or without the help of a selection drug.
Thus, the expression active PCR product or expression vector in accordance with the present invention may additionally comprise a selectable marker.
For the generation of stable cell lines, clones can be selected using various selectable markers, which include, but are not limited to neomycin, blasticidin, puromycin, zeocin, hygromycin, and dihydrofolate reductase (dhfr).

Suitable eukaryotic/mammalian cells (host cells) for all of the above methods are also well known in the art and include, but are not limited to CHO, HEK 293, HeLa, and COS-7 cells.

In one embodiment the above method further comprises the step of translating said protein from said expression active PCR product or expression vector in said eukaryotic cells.

Preferably said protein is selected from the group comprising reporter proteins, therapeutic proteins, antibodies, vaccines, membrane proteins, fusion proteins, blood plasma proteins, cytokines, interferons, growth factors, chemokines, and GPCRs.

The objects of the present invention are also solved by a nucleic acid sequence has a sequence selected from SEQ ID NO: 2, 3, 5, 7, and 9, preferably SEQ ID NO: 9.

The objects of the present invention are also solved by an expression active PCR product or an expression vector comprising the nucleic acid as described above.

The objects of the present invention are further solved by a host cell containing the above expression active PCR product or expression vector.

Also disclosed is a protein produced by the method as described above, wherein said protein is selected from the group comprising reporter proteins, therapeutic proteins, antibodies, vaccines, membrane proteins, fusion proteins, blood plasma proteins, cytokines, interferons, growth factors, chemokines, and GPCRs.

According to the present invention the term "therapeutic proteins" is meant to include proteins used in a pharmaceutical context, such as antibody fragments, immunoglobulin chains, heavy and light chains, Fab fragments, enzymes, growth factors, interferons, cytokines, lymphokines, adhesion molecules, receptors, as well as derivatives or fragments thereof.

According to the present invention reporter proteins (either alone or fused to another protein) are particularly preferred "targets" for the above described method. There are many fluorescent and non-fluorescent reporter proteins including (without being limited to) green fluorescent proteins (GFP), red fluorescence proteins (RFP), yellow fluorescent proteins (YFP), blue and cyanine fluorescent proteins (CFP), luciferase, secreted alkaline phosphatase (SEAP), Chloramphenicol acetyltransferase (CAT), secreted hormone, secreted cytokine, β-galactosidase, and other fluorescent and bioluminescent proteins. The choice of reporter protein depends on the cell line used (endogenous activity), the nature of the experiment (e.g. dynamics of gene expression and transfection efficiency), and the adaptability of the assay to the chosen detection method (Naylor 1999). Several modifications of the reporter proteins themselves have been sought to improve the reporter performance, such as rapid response and magnitude of change, e.g. the use of destabilization elements (Li et al., 1998b; Zhao et al., 1995). Green fluorescent protein (GFP) and other fluorescent proteins are increasingly popular for the non-invasive monitoring of gene expression in living tissues, cells, and in laboratory animals (Naylor 1999). Thus, GFP (and its derivatives, such as EGFP), other fluorescent proteins (see above), as well as fusion proteins comprising a fluorescent protein are even more preferred "target proteins" for the method according to the present invention.

The inventor has surprisingly found that reducing the number of RNase L cleavage sites, preferably UU and UA dinucleotides, in the nucleic acid sequence of a protein to be expressed in a eukaryotic and/or mammalian expression system results in a significantly improved expression and yield of said protein. Without wishing to be bound to a certain theory, it is believed that the reduction of RNase L cleavage sites (i.e. reduced frequency of these sites) results in a nucleic acid sequence, namely an RNA - both primary RNA and mRNA -, that is less prone (i.e. more resistant) to attacks of endogenous RNase L in the eukaryotic host cells, leading to increased mRNA stability and, thus increased protein expression.

Because of the universal concept involved, the present invention can be applied to any sequence. More specifically, the method according to the present invention is applicable to both endogenous and exogenous (recombinant) proteins/genes, to both stably integrated and transiently expressed genes, to proteins/genes that are difficult to express, and also to proteins/genes that are endogenously expressed in low-abundance. It can be applied to both prokaryotic and eukaryotic systems. The approach described herein will lead to a significant reduction of time and costs spent on protein production, and, thus, will allow a more efficient production of proteins/genes used as biopharmaceuticals, such as erythropoietin, growth factors, interferons, insulin, therapeutic and diagnostic antibodies, and protein- or peptide-based vaccines. It is especially useful for the expression of genes/proteins that have been proven to be very difficult to express/produce in large quantities. Examples include, but are not limited to membrane proteins, such as G-protein linked receptors (GPCR), large proteins, antibodies, fusion proteins, protein complexes, vaccines, and blood plasma proteins.
It can also help to significantly improve the expression and performance of reporter proteins, e.g. fluorescent proteins, such as GFP or luciferase, and, thus, increase the sensitivity of methods using such reporter proteins (fluorescence or luminescence microscopy, fluorescence-based microarrays, cell-sorting, etc.).
Furthermore, the approach is particularly practical and simple, since it is applicable to any cell line, such as cell lines used in the biotechnology industry including (but not limited to) hamster CHO1 and HEK293.

The following describes general principles for the step of reducing the number of RNase L cleavage sites according to the present invention:

### Reduction of the number of UU and/or UA dinucleotides in a coding region

Table 2 and Table 3 show the changes that can be made. It is important to note that these modifications are entirely different from the codon usage frequency tables (see for comparison Table 1) that are used to optimize codons for protein expression on the basis of their codon bias (codon usage frequency) in a given organism. The present method is not directed at changing codons on the basis of the codon usage frequency, but in order to reduce the number of RNase L cleavage sites/targets (UU and UA dinucleotides). Moreover, the method according to the present invention is not "organism-dependent"; the number/frequency of UU and/or UA dinucleotides can be reduced in any gene (nucleic acid sequence) from any organism, as long as the corresponding amino acid sequence remains unaltered (see Tables 2 and 3). Furthermore, the reduction of the number of UU and/or UA dinucleotides can also be combined with the classical codon usage optimization for the desired organism (see for example Table 1), possibly resulting in an even more increased expression.
Table 2 shows the codons that harbor UU and/or UA dinucleotides as well as their non-UU/UA-harboring alternative(s). UUU coding for phenylalanine (Phe) can only be changed to UUC, since there is not other codon for phenylalanine that is totally devoid of UU or UA. This is also the case for tyrosine (UAU) that can only be changed to UAC.
Once the changes according to Table 2 are performed, di-triplets that form a UU or UA, i.e. NNU UNN or NNU ANN (with N being any nucleotide) are changed according to Table 3. If there is more than one alternative, preference is given to the more frequently used codon in the respective organism or in highly expressed genes.

Steps for UU and/or UA dinucleotide reduction in coding regions:
1. Change codons according to Table 2.
2. If more than one alternative codon exist, use the more frequently used codon in the desired organism (optional).
3. Change the first codon of the di-triplets that form a UU or UA dinucleotide together (NNU UNN, NNU ANN) according to Table 3.
4. If more than one alternative codon exist, use the more frequently used codon or the strongest for expression in the desired organism (optional, see Tables 4 and 5).
5. Classical codon usage optimization (based on the host cell organism or on a list of codons most frequently used in high expression genes, can be performed optionally and preferably prior to the UU and UA reduction.

### Reduction of the number of UU and/or UA dinucleotides in an intron

Steps for UU and/or UA dinucleotide reduction in introns:
1. Mutate or delete one or two of the two nucleotides in UU/UA dinucleotides: UU or UA to UC, UG, GA, CA, or TA. Alternatively, insert one nucleotide.
2. The entire GC content of the intron should not be more than 80% and the length should not be changed to less than 80% of its original length.
3. Do not change exon-intron boundaries including exon donor and acceptor splice sites and branch points. Avoid disrupting the CT-rich area ranging from the splice acceptor site to the end of the branch point.

### Reduction of the number of UU and/or UA dinucleotides in a 5'UTR

Steps for UU and/or UA dinucleotide reduction in 5'UTRs:
1. Mutate UU or UA to UC, UG, GA, CA, or TA.
2. The entire GC content of the 5'UTR should not be more than 80%.
3. Avoid context sequences near the initiation codon, ATG, since they may harbor translation enhancing sequences, such as kozac.

### Reduction of the number of UU and/or UA dinucleotides in a 3'UTR

Steps for UU and/ UA dinucleotides reduction in 3' UTRs:
1. Mutate or delete one or two of the two nucleotides in UU/UA dinucleotides: UU or UA to UC, UG, GA, CA, or TA. Alternatively, insert one nucleotide.
2. The entire GC content of the 3'UTR should not be more than 80% and the length should not be changed to less than 80% of its original length.
3. Do not change polyA signals such as AAUAAA or AUUAAA and avoid to alter any necessary or accessory sequence elements used for polyadenylation, if found.

### Possible changes in non-coding regions

1. Mutation: NUUN or NUAN to NUSN (where S is G or C, and N is any nucleotide)
2. Insertion: NUUN or NUAN to NUSUN or NUSA (where S is G or C, and N is any nucleotide)
3. Deletion: NUUN to NUS (where S is G or C, and N is any nucleotide)
4. Deletion: NUAN to NAN or NUS (where S is G or C, and N is any nucleotide)

### Tables

**Table 1: Codon frequency in human genes**

| fields: [triplet] [amino acid] [fraction] [frequency: per thousand] ([number]) | | | |
|---|---|---|---|
| UUU F 0.46 17.6 (714298) | UCU S 0.19 15.2 (618711) | UAU Y 0.44 12.2 (495699) | UGU C 0.46 10.6 (430311) |
| UUC F 0.54 20.3 (824692) | UCC S 0.22 17.7 (718892) | UAC Y 0.56 15.3 (622407) | UGC C 0.54 12.6 (513028) |
| UUA L 0.08 7.7 (311881) | UCA S 0.15 12.2 (496448) | UAC * 0.30 1.0 (40285) | UGA * 0.47 1.6(63237) |
| UUG L 0.13 12.9 (525688) | UCG S 0.05 4.4 (179419) | UAG * 0.24 0.8 (32109) | UGG W 1.00 13.2 (535595) |
| | | | |
| CUU L 0.13 13.2 (536515) | CCU P 0.29 17.5 (713233) | CAU H 0.42 10.9 (441711) | CGU R 0.08 4.5 (184609) |
| CUC L 0.20 19.6 (796638) | CCC P 0.32 19.8 (804620) | CAC H 0.58 15.1 (613713) | CGC R 0.18 10.4 (423516) |
| CUA L 0.07 7.2 (290751) | CCA P 0.28 16.9 (688038) | CAA Q 0.27 12.3 (501911) | CGA R 0.11 6.2 (250760) |
| CUG L 0.40 39.6 (1611801) | CCG P 0.11 6.9 (281570) | CAG Q 0.73 34.2 (1391973) | CGG R 0.20 11.4 (464485) |
| | | | |
| AUU I 0.36 16.0 (650473) | ACU T 0.25 13.1 (533609) | AAU N 0.47 17.0 (689701) | AGU S 0.15 12.1 (493429) |
| AUC I 0.47 20.8 (846466) | ACC T 0.36 18.9 (768147) | AAC N 0.53 19.1 (776603) | AGC S 0.24 19.5 (791383) |
| AUA I 0.17 7.5 (304565) | ACA T 0.28 15.1 (614523) | AAA K 0.43 24.4 (993621) | AGA R 0.21 12.2 (494682) |
| AUG M 1.00 22.0 (896005) | ACG T 0.11 6.1 (246105) | AAG K 0.57 31.9 (1295568) | AGG R 0.21 12.0 (486463) |
| | | | |
| GUU V 0.18 11.0 (448607) | GCU A 0.27 18.4 (750096) | GAU D 0.46 21.8 (885429) | GGU G 0.16 10.8 (437126) |
| GUC V 0.24 14.5 (588138) | GCC A 0.40 27.7 (1127679) | GAC D 0.54 25.1 (1020595) | GGC G 0.34 22.2 (903565) |
| GUA V 0.12 7.1 (287712) | GCA A 0.23 15.8 (643471) | GAA E 0.42 29.0 (1177632) | GGA G 0.25 16.5 (669873) |
| GUG V 0.46 28.1 (1143534) | GCG A 0.11 7.4 (299495) | GAG E 0.58 39.6 (1609975) | GGG G 0.25 16.5 (669768) |

| | | | |
|---|---|---|---|
| Source: http://www.kazusa.or.jp | | | |

**Table 2: UU/UA-harboring codons and their non-UU/UA-harboring alternative(s)**

| UU/UA-harboring codon | Amino acid | Alternative codon(s) |
|---|---|---|
| UUU | Phenylalanine (F) | UUC |
| UUA | Leucine (L) | CUC, CUG |
| UUG | Leucine (L) | CUC, CUG |
| CUU | Leucine (L) | CUC, CUG |
| CUA | Leucine (L) | CUC, CUG |
| UAU | Tyrosine (Y) | UAC |
| AUU | Isoleucine (I) | AUC |
| AUA | Isoleucine (I) | AUC |
| GUU | Valine (V) | GUC, GUG |
| GUA | Valine (V) | GUC, GUG |

**Table 3: Di-Triplets forming UU/UA dinucleotides and the corresponding UU/UA-reduced di-triplet(s)**

| **UU/UA-forming di-triplets** | **Amino acid** | **Modified di-triplets** |
|---|---|---|
| UCU UNN | Serine (S) UNN | AGC UNN |
| UCU ANN | Serine (S) ANN | AGCANN |
| CCU UNN | Proline (P) UNN | CCC UNN or CCA UNN |
| CCU ANN | Proline (P) ANN | CCC ANN or CCA ANN |
| ACU UNN | Threonine (T) UNN | ACC UNN or ACA UNN or ACG UNN |
| ACU ANN | Threonine (T) ANN | ACC ANN or ACA ANN or ACG ANN |
| GCU UNN | Alanine (A) UNN | GCC UNN or GCA UNN GCG UNN |
| GCU ANN | Alanine (A) ANN | GCC ANN or GCA ANN GCG ANN |
| CAU UNN | Histidine (H) UNN | CAC UNN |
| CAU ANN | Histidine (H) ANN | CAC ANN |
| AAU UNN | Asparagine (N) UNN | AAC UNN |
| AAU ANN | Asparagine (N) ANN | AAC ANN |
| GAU UNN | Aspartic acid (D) UNN | GAC UNN |
| GAU ANN | Aspartic acid (D) ANN | GACANN |
| UGU UNN | Cysteine (C) UNN | UGC UNN |
| UGU ANN | Cysteine (C) ANN | UGCANN |
| CGU UNN | Arginine (R) UNN | CGC UNN or CGA UNN or AGA UNN or AGG UNN |
| CGU ANN | Arginine (R) ANN | CGC ANN or CGA ANN or AGA ANN or AGG ANN |
| AGU UNN | Serine (S) UNN | AGC UNN or UCC UNN or UCA UNN or UCG UNN |
| AGU ANN | Serine (S) ANN | AGC ANN or UCC ANN or UCA ANN or UCG ANN |
| GGU UNN | Glycine (G) UNN | GGA UNN or GGC UNN or GGG UNN |
| GGU ANN | Glycine (G) UNN | GGA ANN or GGC ANN or GGG ANN |

| | | |
|---|---|---|
| UNN: UUC, UCU, UCC, UCA, UCG, ANN: AUC, AUG, ACU, ACC, ACA, ACG, AAU, AAC, AAA, AAG, AGU, AGC, AGA, AGG | | |

**Table 4: Combination of UU/UA dinucleotide reduction and classical codon optimization (for single codons / triplets)**

| UU/UA codon | Amino Acid | Changed Codons | Codon bias* |
|---|---|---|---|
| UUU | Phenylalanine (F) | UUC | none |
| UUA | Leucine (L) | CUC, CUG | CUG |
| UUG | Leucine (L) | CUC, CUG | CUG |
| CUU | Leucine (L) | CUC, CUG | CUG |
| CUA | Leucine (L) | CUC, CUG | CUG |
| UAU | Tyrosine (Y) | UAC | None |
| AUU | Isoleucine (I) | AUC | None |
| AUA | Isoleucine (I) | AUC | None |
| GUU | Valine (V) | CUC, GUG | GUG |
| GUA | Valine (V) | CUC, GUG | GUG |

| | | | |
|---|---|---|---|
| *Human codon bias as an example of organism codon bias. | | | |

**Table 5: Combination of UU/UA dinucleotide reduction and classical codon optimization (for codon pairs / di-triplets)**

| **WU Di-Triplets** | **Amino Acid** | **Modified Di-Triplets** | **Codon Bias*** |
|---|---|---|---|
| UCU UNN | Serine (S) UNN | AGC UNN or TCG UNN or TCC UNN | AGC UNN |
| UCU ANN | Serine (S) ANN | AGC ANN or TCG UNN or TCC ANN | AGC UNN |
| CCU UNN | Proline (P) UNN | CCC UNN or CCA UNN | CCC UNN |
| CCU ANN | Proline (P) ANN | CCC ANN or CCA ANN | CCC ANN |
| ACU UNN | Threonine (T) UNN | ACC UNN or ACA UNN or ACG UNN | ACC UNN |
| ACU ANN | Threonine (T) ANN | ACC ANN or ACA ANN or ACG ANN | ACC ANN |
| GCU UNN | Alanine (A) UNN | GCC UNN or GCA UNN GCG UNN | GCC UNN |
| GCU ANN | Alanine (A) ANN | GCC ANN or GCA ANN GCG ANN | GCC ANN |
| CAU UNN | Histidine (H) UNN | CAC UNN | None |
| CAU ANN | Histidine (H) ANN | CAC ANN | None |
| AAU UNN | Asparagine (N) UNN | AAC UNN | none |
| AAU ANN | Asparagine (N) ANN | AAC ANN | none |
| GAU UNN | Aspartic acid (D) UNN | GAC UNN | none |
| GAU ANN | Aspartic acid (D) ANN | GACANN | none |
| UGU UNN | Cysteine (C) UNN | UGC UNN | none |
| UGU ANN | Cysteine (C) ANN | UGCANN | none |
| CGU UNN | Arginine (R) UNN | CGC UNN or CGA UNN or AGA UNN or AGG UNN | AGG UNN |
| CGU ANN | Arginine (R) ANN | CGC ANN or CGA ANN or AGA ANN or AGG ANN | AGG ANN |
| AGU UNN | Serine (S) UNN | AGC UNN or UCC UNN or UCA UNN or UCG UNN | AGCUNN |
| AGU ANN | Serine (S) ANN | AGC ANN or UCC ANN or UCA ANN or UCG ANN | AGCANN |
| GGU UNN | Glycine (G) UNN | GGA UNN or GGC UNN or GGG UNN | GGC UNN |
| GGU ANN | Glycine (G) UNN | GGA ANN or GGC ANN or GGG ANN | GGCANN |

| | | | |
|---|---|---|---|
| *Human codon bias as an example of organism codon bias. UNN: UUC, UCU, UCC, UCA, UCG, ANN: AUC, AUG, ACU, ACC, ACA, ACG, AAU, AAC, AAA, AAG, AGU, AGC, AGA, AGG | | | |

Reference is now made to the figures, wherein
**Figure 1** is a graph showing the effect of the reduction of UU and UA dinucleotides on the expression of EGFP in a mammalian expression system in comparison to the wild type sequence,
**Figure 2** is a graph showing the effect of the reduction of UU and UA dinucleotides on the expression of EGFP in a mammalian expression system in comparison to the wild type sequence with simultaneous over-expression of RNase L, and
**Figure 3** is a graph showing the GFP fluorescence in mammalian cells transfected with PCR products harboring a modified UU/UA-reduced EGFP sequence or the wild type EGFP sequence.
**Figure 4** is a graph showing luciferase activity in Hek293 cells transfected with either a wild type or modified (i.e. UU/AG-reduced) firefly luciferase expression vector.
**Figure 5** is a graph showing luciferase activity in Huh7 cells which have been transfected with different PCR products generated from wild type or UU/UA-reduced firefly luciferase expression vector,
**Figure 6** is a graph showing expression of hepatitis B surface antigen in Hek293 cells transfected with a wild type or UU/UA-reduced hepatitis B surface antigen expression vector.
**Figure 7** is a graph showing expression of various green fluorescent proteins in Hek293 cells transfected with MODC-destabilized wildtype and MODC-destabilized UU/UA-reduced green fluorescent proteins.

The invention is now further described by reference to the following examples, which are intended to illustrate, not to limit the scope of the invention.

### EXAMPLE 1: Reduction of UU and/or UA dinucleotides in introns

/ = splicing site
Underlined = consensus functional site
Bold underlined italics = branch point
SEQ ID NO: 1: Wild type rabbit beta globin intron 1 (RBTG1):
SEQ ID NO: 2: UU/UA-reduced RBTG1 (without UU/UA reduction in the CT-rich region):
SEQ ID NO: 3: UU/UA-reduced RBTG1 (with minimal UU/UA reduction in the CT-rich region):

### EXAMPLE 2: Reduction of UU and/or UA dinucleotides in the 3'UTR

. = deletion
Underlined = mutation
Bold underlined = poly A signal
SEQ ID NO: 4: Wild type SV40 3'UTR
SEQ ID NO: 5: UU/UA-reduced SV40 3'UTR
SEQ ID NO: 6: Wild type bovine growth hormone (BGH) 3'UTR
SEQ ID NO: 7: UU/UA-reduced BGH 3'UTR

### EXAMPLE 3: Reduction of UU and/or UA dinucleotides in the codin region

SEQ ID NO: 8: Wild type enhanced green fluorescent protein (EGFP)
SEQ ID NO: 9: UU/UA-reduced EGFP ("SuperGFP")
**SEQ ID NO: 10**
   **WILD TYPE GFP SEQUENCE FROM *MONTASTREA CAVERNOSA***
**SEQ ID NO: 11**
   **MONSTER-OM: MODIFIED TYPE OF SEQ ID NO: 10 BY REDUCING THE NUMBER OF UU/UA DINUCLEOTIDES**
**SEQ ID NO: 12**
   ***CLAVULARIA* SPECIES - WILD TYPE SEQUENCE**
**SEQ ID NO.: 13**
   ***CLAVULARIA* SPECIES-OM: MODIFIED (UU/UA-reduced)**
**SEQ ID NO: 14**
   **FIREFLY LUCIFERASE +: WILD TYPE SEQUENCE:**
**SEQ ID NO: 15**
   **LUC+DU (*Superluciferase*): (UU/UA-reduced)**
**SEQ ID NO: 16**
   **Firefly LUC2 luciferase wildtype:**
**SEQ ID NO: 17**
   **LUC2OM: LUC2 modified *SuperLuciferase2* (UU/CTA-reduced)**
**SEQ ID NO: 18**
   ***Puntellina Plumate* (GFP) wild type:**
**SEQ ID NO: 19**
   ***Puntellina Plumate** (GFP)* **:modifed sequence (UU/UA-reduced)**
**SEQ ID NO: 20**
   **Red Fluorescent protein from Discosoma wild type sequence**
**SEQ ID NO: 21**
   **Red Fluorescent protein modified sequence**
**SEQ ID NO: 22**
   **Hepatitis B surface antigen wild type, adr hepatitis B virus strain:**
**SEQ ID NO: 23**
   **HBSAGOM: Hepatitis B surface antigen modified sequence:**
**SEQ ID NO: 24**
   **HBSAGM: HBSAGOM: Hepatitis B surface antigen modified sequence 2**

This sequence includes both an VULVA reduction as well as a humanization.
**SEQ ID NO: 25**
   **IFN-ALPHA, HUMAN**
**SEQ ID NO: 26**
   **IFN-ALPHAOM: modified sequence**
**SEQ ID NO: 27**
   **CSF3M Colony stimulating factor wild type sequence, human:**
**SEQ ID NO: 28**
   **CSF3M Colony stimulating factor modified sequence,:**
**SEQ ID NO: 29 MODC wild type sequence (mouse ornithine decarboxylase)**
**SEQ ID NO: 30 MODC modified sequence:**

Destabilized and sequence modified reporters:

### EXAMPLES OF destabilized and modified REPORTER SEQUENCES:

**SEQ ID NO: 31**
   ***MONTASTRAEA CAVERNOSA*** **Bold in MODC seqeuence.**
**SEO ID NO: 32**
   ***Clavulariidae Clavularia* -OM: MODIFIED**
**SEQ ID NO: 33**
   **Firefly LUC+DU (*Superluciferase*):**
**SEO ID NO: 34**
   **Firefly LUC2OM: LUC2 modified *SuperLuciferase2***
**SEQ ID NO: 35**
   ***Puntellina Plumate* GFP: modified sequences**
**SEQ ID NO: 36**
   **Red Fluorescent protein modified sequence**

### EXAMPLE 4: In vivo analysis of UU/UA-reduced EGFP

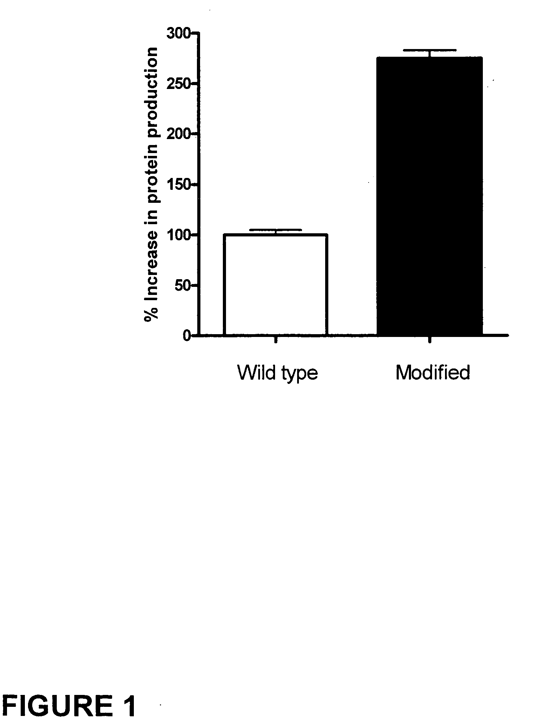

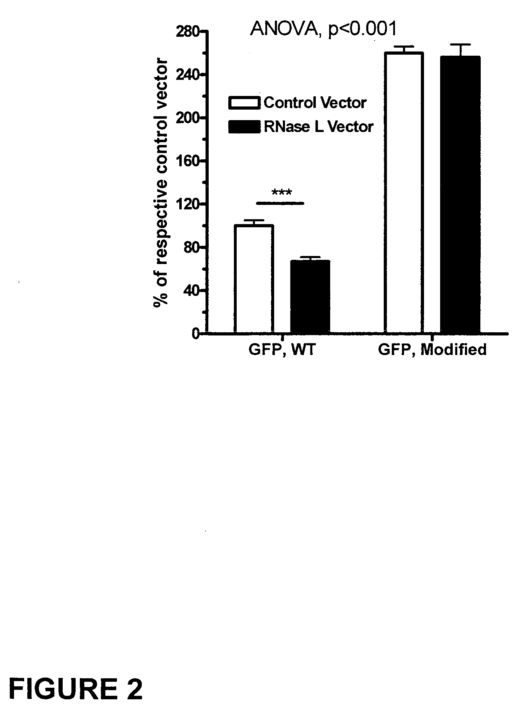

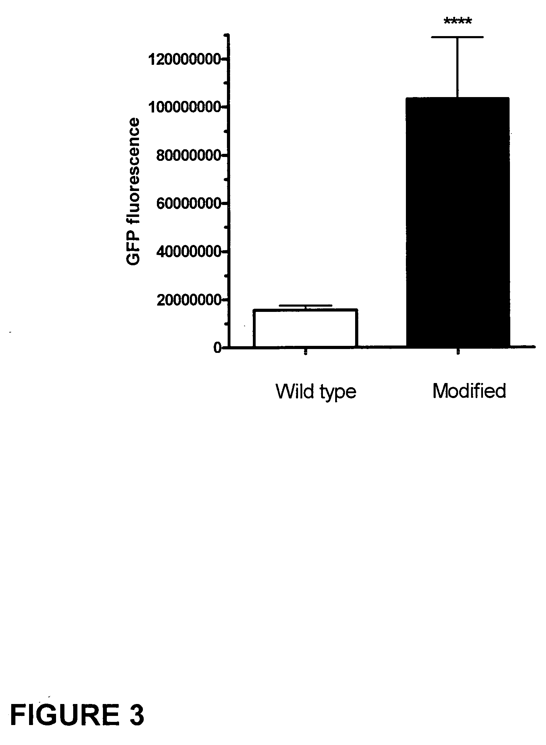

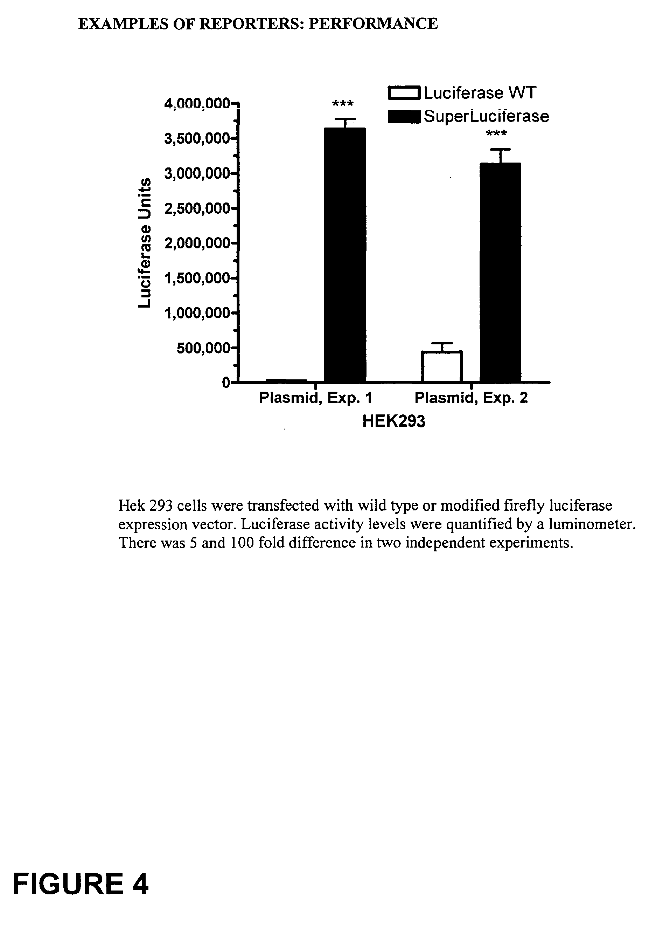

(1) The modified EGFP sequence was custom synthesized by a gene synthesis company and supplied contained in a pUC19 vector with flanking SalI and BamHI sites. 10 µg of the vector were digested with 10 units of SalI in a buffer containing 0.1 µg/ml BSA for 1 hr at 37°C, followed by digestion with BamHI in BamHI buffer for an additional hour at 37°C. The digested DNA was extracted using the phenol-chloroform method, followed by ethanol precipitation. The synthetic EGFP-coding region was ligated into an expression vector, which had a CMV promoter and a BGH 3'UTR and had been digested with the same restriction enzymes (SalI and XbaI) and purified by phenol-chloroform extraction, followed by ethanol precipitation. Cloning of the EGFP-DNA into the expression vector was performed using the following ligation reaction: 30 %g of digested vector DNA were mixed with 90 µg of digested EGFP-DNA in a 10 µl reaction containing T4 DNA ligase. The ligated products were used to transform DH5α competent *E. coli* cells followed by expansion of the resulting colonies in a bacterial culture medium. The recombinant DNA was extracted using a Qiagen plasmid purification kit (Qiagen, Germany). The sizes of the vectors harboring the inserts were verified using gel electrophoresis. The resultant expression vector with the modified UU/UA-reduced coding region along with a vector containing the wild type DNA were used for functional studies to confirm the expression of the encoded protein. HEK293 cells were grown at standard culture conditions (37°C, 5% CO₂) in DMEM medium supplemented with 10% FBS and antibiotics (Invitrogen). 2.5 x 10⁴ cells per well in 96-well plates were transfected with 100 ng of the vector with the modified UU/UA-reduced coding region of EGFP or the vector containing the wild-type EGFP-DNA. Transfections were performed in serum-free medium using Lipofectamine 2000 (Invitrogen) for 5 h, followed by replacing the medium with serum-supplemented medium. After approximately 24 or 48 hours, the plates were imaged and quantified using a BD high-content imager. Quantification was performed with a Proxcell imaging algorithm.
   The data clearly shows that the use of the UU/UA-reduced coding region of EGFP allows a significantly (2.5 to 3-fold) higher expression of EGFP in eukaryotic cells than that of the wild type EGFP-DNA (**Figure 1**).
(2) HEK293 cells were grown at standard culture conditions (37°C, 5% CO₂) in DMEM medium supplemented with 10% FBS and antibiotics (Invitrogen). 2.5 x 10⁴ cells per well in 96-well plates were transfected with 100 ng of the vector with the modified UU/UA-reduced coding region of EGFP or the vector containing the wild-type EGFP-DNA. The cells were also co-transfected with either an empty control vector (pcDNA 3.1) or a RNase L vector (pcDNA 31). Transfections were performed in serum-free medium using Lipofectamine 2000 (Invitrogen) for 5 h, followed by replacing the medium with serum-supplemented medium. After approximately 24 or 48 hours, the plates were imaged and quantified using a BD high-content imager. Quantification was performed with a Proxcell imaging algorithm.
   The data shows that - in comparison/contrast to the wild type EGFP sequence - the use of the modified EGFP sequence resulted in higher expression, which was not constrained by the co-expression of RNase L (**Figure 2**).
(3) Expression active PCR products were generated by using primers in which the forward (5') primers were complementary to the beginning of the CMV promoter region or a sequence upstream of the CMV promoter, while the reverse (3') primers were complementary to the BGH poly A site or a sequence downstream of this site. The PCR was carried out using a mixture of Taq and Pfu polymerase in a 100 µl reaction with the following cycle conditions: - 95°C for 12 min (to activate hot start polymerases), - 32 cycles of: 94°C, 1 min; 52°C, 1 min; 72°C, 4 min, and a final extension at 72°C for 7 min. The PCR products were purified using Qiagen PCR purification columns to eliminate the primers, small PCR products, buffer, and enzymes, and eluted in sterile water. HEK293 cells were grown at standard culture conditions (37°C, 5% CO₂) in DMEM medium supplemented with 10% FBS and antibiotics (Invitrogen). 2.5 x 10⁴ cells per well in 96-well plates were transfected with 100 ng of purified PCR products generated from the EGFP expression vector with the wild type or with the modified sequence. Transfections were performed in serum-free medium using Lipofectamine 2000 (Invitrogen) for 5 h, followed by replacing the medium with serum-supplemented medium. After approximately 24 or 48 hours, the plates were imaged and quantified using a BD high-content imager. Quantification was performed with a Proxcell imaging algorithm.
   The data shows that PCR products harboring the UU/UA-reduced coding region of EGFP led to higher expression of EGFP (5 to 10-fold increase) than those harboring the wild type sequence (**Figure 3**).
(4) Using the same methodology as described in (2) and (3), Hek293 cells were transfected with wild type or UU/UA-reduced firefly luciferase expression vector ("Superluciferase", SEQ ID NO: 15). The luciferase activity levels were quantified by a 1,L111111V111GtG1. The data show that, within two independent experiments, there was an approximately 5- and approximately 100-fold difference (**Figure 4**).

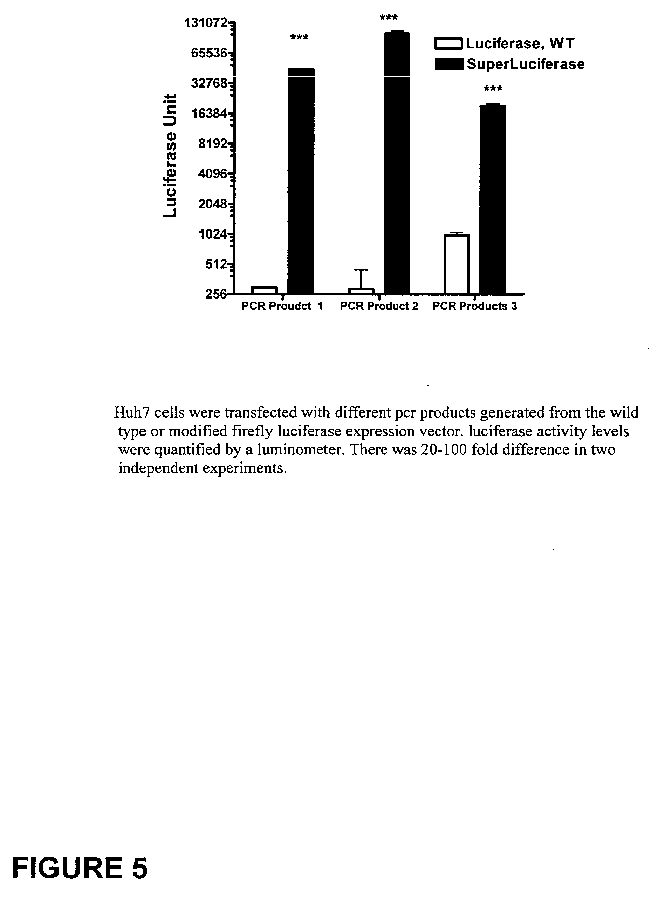

Likewise, Huh7 cells were transfected with different PCR products generated in accordance with the methodology outlined in (3) above, from the wild type or modified firefly luciferase expression vector ("Superluciferase"; SEQ ID NO: 15). The luciferase activity levels were, again, quantified by a luminometer. The data show that PCR products harbouring the UU/UA-reduced coding region of superluciferase led to a substantially higher expression of luciferase (20-100-fold increase) than those harbouring the wildtype sequence (**Figure 5**). This demonstrates the method in accordance with the present invention works with a verity of variety of reporter proteins.

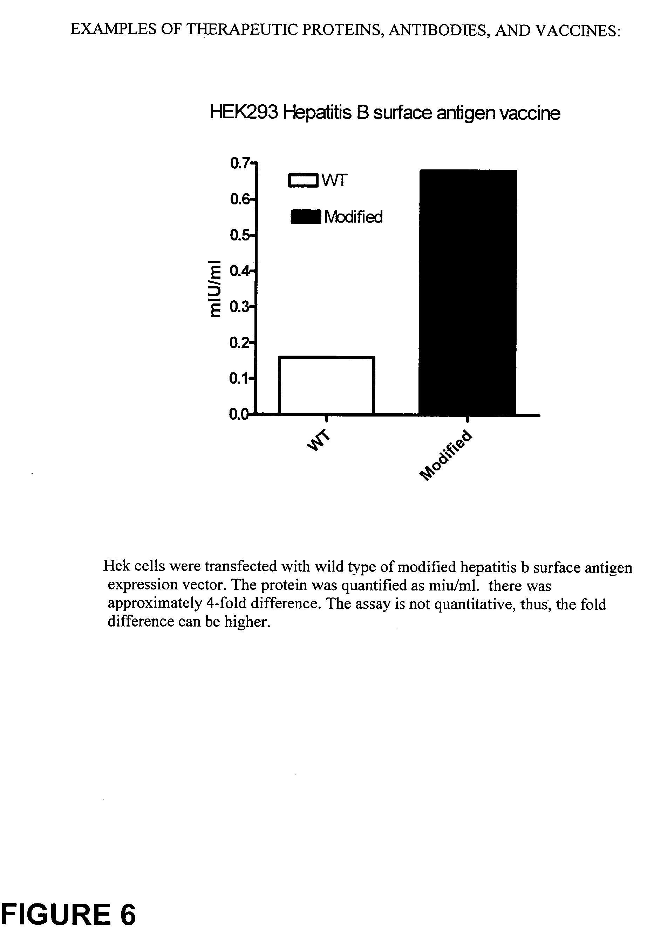

Likewise, Hek293 cells were transfected, using the same methodology as in (2), above, with a wild type or UU/UA-reduced hepatitis B surface (SEQ ID NO: 23) antigen expression vector. The expressed protein was quantified as mIU/ml. There was approximately a 4-fold difference (**Figure 6**), but it is likely that this may be even higher in independent experiments.

This is an example that the method in accordance with the present invention also works with therapeutic proteins, antibodies and vaccines which have been modified, i.e. their coding sequence has been UU/UA-reduced, and this leads to a substantial increase in expression.
(5) In order to reflect the transcriptional changes and the subsequent effects on reported protein levels, protein-destabilizing amino acid regions that include a PEST sequence (= peptide sequence which is rich in proline, glutamic acid, serine and threonine) have been used to reduce the half-life of various reporter proteins. PEST sequences are associated with proteins that have a short intracellular half-life. Li et al. (J. Biol. Chem., 1998, 273, pp. 34970-34975) describe the use of a PEST sequence of MODC to destabilize the EGFP, and Leclerc et al. (Biotechniques, 2000, 29, pp. 590-591, pp. 594-596 used a PEST sequence to reduce the protein half-life of firefly luciferase.

Using such MODC-domain (mouse ornithine decarboxylase), more specifically, amino acids 422-461 of the degradation domain of the highly unstable MODC, the present inventor rendered a number of reporter genes unstable by fusing them with the afore-mentioned MODC domain. Moreover, the present inventor modified such fusions by reducing the number of UU/UA dinucleotides in both the reporter gene and the MODC domain in accordance with the present invention with respect to EGFP from Aequorea Victoria, Montastrea Cavernosa, Clavularia and Puntelina Plumate. The number of UU/UA dinucleotides waas reduced both in the EGFP-part and the MODC part of the fusion.

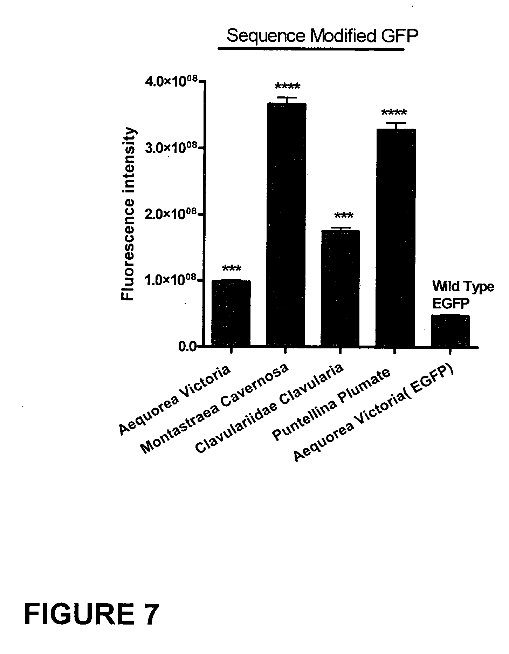

The MODC domain was amplified from genomic DNA of mouse fibroblasts using specific primers that contain EcoRI and BamHI sites in the forward and reverse primer, respectively. The amplified cDNA was cloned in frame with the GFP coding region using the same restriction sites. Hek293 cells were transfected with destabilized GFPs as indicated in figure 7. The fluorescence intensity was quantitated by imaging apparatus and software. Compared to destabilized wildtype EGFP from Aequorea Victoria (i.e. the point of reference was wildtype EGFP, SEQ ID NO: 8, fused to wildtype MODC, SEQ ID NO: 29), there was a two-fold increase in fluorescence from modified Aequorea Victoria, an 8-fold increase from modified Montastrea Cavernosa green fluorescent protein, a 4-fold increase from modified Clavularia green fluorescent protein and a 7-fold increase from modified Puntelina Plumate green fluorescent protein (see figure 7). The term "modified" here means "UU/UA reduced and fused with MODC which itself has also been UU/UA reduced".

Consequently, this shows that the present invention also works in situations where expression signals normally are weaker, and improves the fold-increase in expression in such situations.

### REFERENCES

Al-Zoghaibi F., T. Ashour, W. Al-Ahmadi, H. Abulleef, O. Demirkaya, and K.S.A. Khabar. 2007. Bioinformatics and experimental derivation of an efficient hybrid 3' untranslated region and use in expression active linear DNA with minimum poly(A) regions. Gene 391: 130-139.
Bakheet, T., B.R. Williams, and K.S. Khabar. 2006. ARED 3.0: the large and diverse AU-rich transcriptome. Nucleic Acids Res 34: D111-114.
Barreau, C., L. Paillard, and H.B. Osborne. 2005. AU-rich elements and associated factors: are there unifying principles? Nucleic Acids Res 33: 7138-7150.
Bisbal, C., M. Silhol, H. Laubenthal, T. Kaluza, G. Carnac, L. Milligan, F. Le Roy, and T. Salehzada. 2000. The 2'-5' oligoadenylate/RNase L/RNase L inhibitor pathway regulates both MyoD mRNA stability and muscle cell differentiation. Mol Cell Biol 20: 4959-4969.
Chandrasekaran, K., Z. Mehrabian, X.L. Li, and B. Hassel. 2004. RNase-L regulates the stability of mitochondrial DNA-encoded mRNAs in mouse embryo fibroblasts. Biochem Biophys Res Commun 325: 18-23.
Dong, B., M. Niwa, P. Walter, and R.H. Silverman. 2001. Basis for regulated RNA cleavage by functional analysis of RNase L and Irelp. Rna 7: 361-373.
Foecking, M.K., and Hofstetter, H. 1986. Powerful and versatile enhancer-promoter unit for mammalian expression vectors. Gene 45: 101-105.
Han, J. Q., G. Wroblewski, Z. Xu, R. H. Silverman, and D. J. Barton. 2004. Sensitivity of hepatitis C virus RNA to the antiviral enzyme ribonuclease L is determined by a subset of efficient cleavage sites. J Interferon Cytokine Res 24: 664-76.
Hassel, B.A., A. Zhou, C. Sotomayor, A. Maran, and R.H. Silverman. 1993. A dominant negative mutant of 2-5A-dependent RNase suppresses antiproliferative and antiviral effects of interferon. Embo J 12: 3297-3304.
Khabar, K.S., Y.M. Siddiqui, F. al-Zoghaibi, L. al-Haj, M. Dhalla, A. Zhou, B. Dong, M. Whitmore, J. Paranjape, M.N. Al-Ahdal et al. 2003a. RNase L mediates transient control of the interferon response through modulation of the double-stranded RNA-dependent protein kinase PKR. J Biol Chem 278: 20124-20132.
Khabar, K.S., Y.M. Siddiqui, F. al-Zoghaibi, L. al-Haj, M. Dhalla, A. Zhou, B. Dong, M. Whitmore, J. Paranjape, M.N. Al-Ahdal et al. 2003b. RNase L mediates transient control of the interferon response through modulation of the double-stranded RNA-dependent protein kinase PKR. J Biol Chem 278: 20124-20132.
Kobayashi, M., Tanaka, A., Hayashi, Y., and Shimanmura, S. 1997. The CMV enhancer stimulates expression of foreign genes from the human EF-1 alpha promoter. Anal Biochem 247: 179-181.
Lai, W.S., J.S. Parker, S.F. Grissom, D.J. Stumpo, and P.J. Blackshear. 2006. Novel mRNA targets for tristetraprolin (TTP) identified by global analysis of stabilized transcripts in TTP-deficient fibroblasts. Mol Cell Biol 26: 9196-9208.
Lal, A., K. Mazan-Mamczarz, T. Kawai, X. Yang, J.L. Martindale, and M. Gorospe. 2004. Concurrent versus individual binding of HuR and AUF1 to common labile target mRNAs. Embo J 23: 3092-3102.
Legendre M, Gautheret D. 2003 Sequence determinants in human polyadenylation site selection. BMC Genomics. 4: 7.
Le Roy, F., C. Bisbal, M. Silhol, C. Martinand, B. Lebleu, and T. Salehzada. 2001. The 2-5A/RNase L/RNase L inhibitor (RLI) [correction of (RNI)] pathway regulates mitochondrial mRNAs stability in interferon alpha-treated H9 cells. J Biol Chem 276: 48473-48482.
Li, X., J.A. Blackford, and B.A. Hassel. 1998a. RNase L Mediates the Antiviral Effect of Interferon through a Selective Reduction in Viral RNA during Encephalomyocarditis Virus Infection. Journal of Virology 72: 2752-2759.
Li, X., X. Zhao, Y. Fang, X. Jiang, T. Duong, C. Fan, C.C. Huang, and S.R. Kain. 1998b. Generation of destabilized green fluorescent protein as a transcription reporter. J Biol Chem 273: 34970-34975.
Li, X.L., J.A. Blackford, C.S. Judge, M. Liu, W. Xiao, D.V. Kalvakolanu, and B.A. Hassel. 2000. RNase-L-dependent destabilization of interferon-induced mRNAs. A role for the 2-5A system in attenuation of the interferon response. J Biol Chem 275: 8880-8888.
Lopez de Silanes, I., S. Galban, J.L. Martindale, X. Yang, K. Mazan-Mamczarz, F.E. Indig, G. Falco, M. Zhan, and M. Gorospe. 2005. Identification and functional outcome of mRNAs associated with RNA-binding protein TIA-1. Mol Cell Biol 25: 9520-9531.
Naylor, L.H. 1999. Reporter gene technology: the future looks bright. Biochem Pharmacol 58: 749-757.
Wrechester, D. H., J. W. McCauley, J. J. Skehel, and I. M. Kerr. 1981. Interferon action: sequence specificity of the ppp(A2'p)nA-dependent ribonuclease. Nature 289: 414-7.
Wreschner, D. H., James, T. C., Silverman, R. H., and Kerr, I. M. (1981). Nucleic Acids Res 9: 1571-1581.
Zhao, S., S.L. Ooi, and A.B. Pardee. 1995. New primer strategy improves precision of differential display. Biotechniques 18: 842-846, 848, 850.
Zhou, A., J. Paranjape, T.L. Brown, H. Nie, S. Naik, B. Dong, A. Chang, B. Trapp, R. Fairchild, C. Colmenares et al. 1997. Interferon action and apoptosis are defective in mice devoid of 2',5'- oligoadenylate-dependent RNase L. Embo J 16: 6355-6363.

## Claims

1. Method for increasing the expression of a protein in eukaryotic cells, said method comprising the step of reducing the number of RNase L cleavage sites in the nucleic acid sequence of said protein.

2. Method according to claim 1, wherein said number of RNase L cleavage sites is reduced by at least 10%, preferably at least 25%, more preferably at least 50%.

3. Method according to claim 1 or 2, wherein said cleavage sites are UU and/or UA dinucleotides.

4. Method according to any of the foregoing claims, wherein said step of reducing the number of RNase L cleavage sites reduces said number in the coding region of said nucleic acid sequence, wherein, preferably, said step of reducing the number of RNase L cleavage sites in said nucleic acid sequence is performed without altering the amino acid sequence of said protein.

5. Method according to claim 4, wherein in said step of reducing the number of RNase L cleavage sites, which is performed without altering the amino acid sequence of said protein, a codon comprising a UU and/or UA dinucleotide is exchanged for an alternative codon not comprising a UU and/or UA dinucleotide and coding for the same amino acid, or wherein in said step of reducing the number of RNase L cleavage sites, which is performed without altering the amino acid sequence of said protein, at least one codon of an adjacent pair of codons comprising a UU and/or UA dinucleotide is exchanged for an alternative codon coding for the same amino acid so that said adjacent pair of codons does no longer comprise a UU and/or UA dinucleotide.

6. Method according to claim 5, wherein said alternative codon is the more frequently used codon in said eukaryotic cells.

7. Method according to any of claims 1 to 3, wherein said step of reducing the number of RNase L cleavage sites reduces said number in the non-coding region of said nucleic acid sequence, wherein, preferably, said non-coding region is a 5'UTR, a 3'UTR, or an intron.

8. Method according to claim 7, wherein said step of reducing the number of RNase L cleavage sites is performed by mutation, deletion, or insertion of nucleotides.

9. Method according to any of the foregoing claims further comprising the step of codon optimization prior to said step of reducing the number of RNase L cleavage sites.

10. Method according to any of the foregoing claims further comprising the step of transfecting said nucleic acid sequence of said protein into said eukaryotic cells in form of an expression active PCR product or contained in an expression vector after said step of reducing the number of RNase L cleavage sites, wherein, preferably, said method further comprises the step of translating said protein from said expression active PCR product or expression vector in said eukaryotic cells.

11. Method according to any of the foregoing claims, wherein said protein is selected from the group comprising reporter proteins, therapeutic proteins, antibodies, vaccines, membrane proteins, fusion proteins, blood plasma proteins, cytokines, interferons, growth factors, chemokines, and GPCRs.

12. Nucleic acid sequence selected from SEQ ID NO: 2, 3, 5, 7, 9, 11, 13, 15, 17, 19, 23, 24, 26, 28, 30-36.

13. Expression active PCR product or expression vector comprising the nucleic acid sequence according to claim 12.

14. Host cell of any organism containing the expression active PCR product or expression vector according to claim 13.

## Patentansprüche

1. Verfahren zum Erhöhen der Expression eines Proteins in eukaryotischen Zellen, wobei das Verfahren den Schritt des Reduzierens der Anzahl an RNase-L-Spaltstellen in der Nukleinsäuresequenz des Proteins umfasst.

2. Verfahren nach Anspruch 1, wobei die Anzahl an RNase-L-Spaltstellen um wenigstens 10 %, bevorzugt wenigstens 25 %, bevorzugter wenigstens 50 %, reduziert wird.

3. Verfahren nach Anspruch 1 oder 2, wobei die Spaltstellen UU- und/oder UA-Dinukleotide sind.

4. Verfahren nach einem der vorangehenden Ansprüche, wobei der Schritt des Reduzierens der Anzahl an RNase-L-Spaltstellen die Anzahl in der kodierenden Region der Nukleinsäuresequenz reduziert, wobei bevorzugt der Schritt des Reduzierens der Anzahl an RNase-L-Spaltstellen in der Nukleinsäuresequenz durchgeführt wird, ohne die Aminosäuresequenz des Proteins zu verändern.

5. Verfahren nach Anspruch 4, wobei bei dem Schritt des Reduzierens der Anzahl an RNase-L-Spaltstellen, welcher durchgeführt wird, ohne die Aminosäuresequenz des Proteins zu verändern, ein Codon, umfassend ein UU- und/oder UA-Dinukleotid, gegen ein alternatives Codon ausgetauscht wird, das kein UU- und/oder UA-Dinukleotid umfasst und für dieselbe Aminosäure kodiert, oder wobei bei dem Schritt des Reduzierens der Anzahl an RNase-L-Spaltstellen, der durchgeführt wird, ohne die Aminosäuresequenz des Proteins zu verändern, wenigstens ein Codon eines angrenzenden Paares von Codons, umfassend ein UU-und/oder UA-Dinukleotid, gegen ein alternatives Codon ausgetauscht wird, das für dieselbe Aminosäure kodiert, so dass das angrenzende Paar von Codons nicht länger ein UU- und/oder UA-Dinukleotid umfasst.

6. Verfahren nach Anspruch 5, wobei das alternative Codon das häufiger verwendete Codon in den eukaryotischen Zellen ist.

7. Verfahren nach einem der Ansprüche 1-3, wobei der Schritt des Reduzierens der Anzahl an RNase-L-Spaltstellen die Anzahl in der nicht-kodierenden Region der Nukleinsäuresequenz reduziert, wobei bevorzugt die nicht-kodierende Region ein 5' UTR, ein 3' UTR oder ein Intron ist.

8. Verfahren nach Anspruch 7, wobei der Schritt des Reduzierens der Anzahl an RNase-L-Spaltstellen mittels Mutation, Deletion oder Insertion von Nukleotiden durchgeführt wird.

9. Verfahren nach einem der vorangehenden Ansprüche, weiterhin umfassend den Schritt der Codon-Optimierung vor dem Schritt des Reduzierens der Anzahl an RNase-L-Spaltstellen.

10. Verfahren nach einem der vorangehenden Ansprüche, weiterhin umfassend den Schritt des Transfizierens der Nukleinsäuresequenz des Proteins in die eukaryotischen Zellen in Form eines expressionsaktiven PCR-Produkts oder enthalten in einem Expressionsvektor nach dem Schritt des Reduzierens der Anzahl an RNase-L-Spaltstellen, wobei bevorzugt das Verfahren weiterhin den Schritt des Translatierens des Proteins von dem expressionsaktiven PCR-Produkt oder von dem Expressionsvektor in den eukaryotischen Zellen umfasst.

11. Verfahren nach einem der vorangehenden Ansprüche, wobei das Protein ausgewählt ist aus der Gruppe, umfassend Reporterproteine, therapeutische Proteine, Antikörper, Vakzine, Membranproteine, Fusionsproteine, Blutplasmaproteine, Cytokine, Interferone, Wachstumsfaktoren, Chemokine und GPCRs.

12. Nukleinsäuresequenz, ausgewählt aus SEQ ID NO: 2, 3, 5, 7, 9, 11, 13, 15, 17, 19, 23, 24, 26, 28, 30-36.

13. Expressionsaktives PCR-Produkt oder Expressionsvektor, umfassend die Nukleinsäuresequenz nach Anspruch 12.

14. Wirtszelle eines Organismus, enthaltend das expressionsaktive PCR-Produkt oder den Expressionsvektor nach Anspruch 13.

## Revendications

1. Procédé permettant d'augmenter l'expression d'une protéine dans des cellules eucaryotes, ledit procédé comprenant l'étape de réduction du nombre de sites de clivage de l'ARNase L dans la séquence d'acide nucléique de ladite protéine.

2. Procédé selon la revendication 1, dans lequel ledit nombre de sites de clivage de l'ARNase L est réduit d'au moins 10 %, de préférence d'au moins 25 %, et de manière davantage préférée d'au moins 50 %.

3. Procédé selon la revendication 1 ou 2, dans lequel lesdits sites de clivage sont les dinucléotides UU et/ou UA.

4. Procédé selon l'une quelconque des revendications précédentes, dans lequel ladite étape de réduction du nombre de sites de clivage de l'ARNase L réduit ledit nombre dans la région codante de ladite séquence d'acide nucléique, dans lequel, de préférence, ladite étape de réduction du nombre de sites de clivage de l'ARNase L dans ladite séquence d'acide nucléique est réalisée sans modifier la séquence d'acides aminés de ladite protéine.

5. Procédé selon la revendication 4, dans lequel, dans ladite étape de réduction du nombre de sites de clivage de l'ARNase L, qui est réalisée sans modifier la séquence d'acides aminés de ladite protéine, comprend l'échange d'un codon comprenant un dinucléotide UU et/ou UA par un codon alternatif ne comprenant pas de dinucléotide UU et/ou UA et codant pour le même acide aminé, ou dans lequel, dans ladite étape de réduction du nombre de sites de clivage de l'ARNase L, qui est réalisée sans modifier la séquence d'acides aminés de ladite protéine, comprend l'échange d'au moins un codon d'une paire adjacente de codons comprenant un dinucléotide UU et/ou par un codon alternatif codant pour le même acide aminé de façon que ladite paire adjacente de codons ne comprend plus aucun dinucléotide UU et/ou UA.

6. Procédé selon la revendication 5, dans lequel ledit codon alternatif est le codon le plus fréquemment utilisé dans lesdites cellules eucaryotes.

7. Procédé selon l'une quelconque des revendications 1 à 3, dans lequel ladite étape de réduction du nombre de sites de clivage de l'ARNase L réduit ledit nombre de ladite séquence d'acide nucléique dans la région non codante, dans lequel, de préférence, ladite région non codante est une UTR en 5', une UTR en 3' ou un intron.

8. Procédé selon la revendication 7, dans lequel ladite étape de réduction du nombre de sites de clivage de l'ARNase L est réalisée par mutation, délétion ou insertion de nucléotides.

9. Procédé selon l'une quelconque des revendications précédentes, comprenant en outre l'étape d'optimisation des codons avant ladite étape de réduction du nombre de sites de clivage de l'ARNase L.

10. Procédé selon l'une quelconque des revendications précédentes, comprenant en outre l'étape de transfection de ladite séquence d'acide nucléique de ladite protéine dans lesdites cellules eucaryotes sous la forme d'un produit PCR actif d'expression ou contenue dans un vecteur d'expression après ladite étape de réduction du nombre de sites de clivage de l'ARNase L, dans lequel, de préférence, ledit procédé comprend en outre l'étape de traduction de ladite protéine à partir dudit produit PCR actif d'expression ou dudit vecteur d'expression dans lesdites cellules eucaryotes.

11. Procédé selon l'une quelconque des revendications précédentes, dans lequel ladite protéine est choisie dans le groupe comprenant les protéines de rapporteur, les protéines thérapeutiques, les anticorps, les vaccins, les protéines membranaires, les protéines de fusion, les protéines du plasma sanguin, les cytokines, les interférons, les facteurs de croissance, les chimiokines et le GPCR.

12. Séquence d'acide nucléique choisie parmi SEQ ID NO : 2, 3, 5, 7, 9, 11, 13, 15, 17, 19, 23, 24, 26, 28, 30 à 36.

13. Produit PCR actif d'expression ou vecteur d'expression, comprenant la séquence d'acide nucléique selon la revendication 12.

14. Cellule hôte de n'importe quel organisme, contenant le produit PCR actif d'expression ou le vecteur d'expression selon la revendication 13.


### SEQUENCE LISTING

<110> King Faisal Specialist Hospital & Research Centre
<120> A method for increasing protein expression in cells
<130> K30371PCT
<160> 36
<170> PatentIn version 3.5
<210> 1
   <211> 175
   <212> DNA
   <213> Oryctolagus cuniculus
<400> 1
   ggtgaggccg agtttggtaa gtatcctttt tacagcacaa cttaatgaga cagatagaaa      60
   ctgaccggtg ggagtctgcg gccgcagtct tgtagaaaca gagtagtcgc ctgcttttct     120
   gccaggtgct gacttctctc cccttctctt ttttcctttt ctcaggttgg tgtcg          175
<210> 2
   <211> 173
   <212> DNA
   <213> Artificial Sequence
<220>
   <223> Reduced number of UU and/or UA dinucleotides
<400> 2
   ggtgaggccg agtttggtaa gtgtcctctg aacagcacaa ctgaatgaga cagaagaaac     60
   tgaccggtgg gagtctgcgg ccgcagtctg tagaaacaga gtagtcgcct gcttttctgc    120
   caggtgctga cttctctccc cttctctttt ttccttttct caggttggtg tcg           173
<210> 3
   <211> 173
   <212> DNA
   <213> Artificial Sequence
<220>
   <223> Reduced number of UU and/or UA dinucleotides
<400> 3
   ggtgaggccg agtttggtaa gtgtcctctg aacagcacaa ctgaatgaga cagaagaaac     60
   tgaccggtgg gagtctgcgg ccgcagtctg tagaaacaga gtagtcgcct gtcttctgcc    120
   aggtgctgac tctctctccc cttctctctc ttcctcttct caggttggtg tcg           173
<210> 4
   <211> 126
   <212> DNA
   <213> Simian virus 40
<400> 4
   tgaatgcaat tgttgttgtt aacttgttta ttgcagctta taatggttac aaataaagca     60
   atagcatcac aaatttcaca aataaagcat ttttttcact gcattctagt tgtggtttgt    120
   ccaaac                                                               126
<210> 5
   <211> 118
   <212> DNA
   <213> Artificial Sequence
<220>
   <223> Reduced number of UU and/or UA dinucleotides
<400> 5
   tgaatgcaat gtgcgtcaac tgtctgtctg cagctcacaa tggttacaaa taaagcaatg     60
   catcacaaat ctcacaaatc aagcatctgt cactgcatct agtgtggtct gtccaaac     118
<210> 6
   <211> 245
   <212> DNA
   <213> Bos taurus
<400> 6
   tctagagatc tgtgtgttgg ttttttgtgg atctgctgtg ccttctagtt gccagccatc       60
   tgttgtttgc cctccccccg tgccttcctt gaccctggaa ggtgccactc ccactgtcct      120
   ttcctaataa aatgaggaaa ttgcatcgca ttgtctgagt aggtgtcatt ctattctggg      180
   gggtggggtg gggcagcaca gcaaggggga ggattgggaa gacaatagca ggcatgctgc      240
   ttaag                                                                  245
<210> 7
   <211> 237
   <212> DNA
   <213> Artificial Sequence
<220>
   <223> Reduced number of UU and/or UA dinucleotides
<400> 7
   tctagagatc tgtgtgttgg tctgtgtgga tctgctgtgc ctctagtgcc agccatctgt       60
   gtctgcccct cccccgtgcc tcctgaccct ggaaggtgcc actcccactg tcctgtccta      120
   ataaaatgag gaaatgcatc gcatgtctga gtggtgtcat ctctatcctg gggggtgggg      180
   tggggcagca cagcaagggg gaggatctgg gaagacaatg caggcatgct gcttaag         237
<210> 8
   <211> 717
   <212> DNA
   <213> Artificial Sequence
<220>
   <223> Enhanced green fluorescent protein
<400> 8
   atggctagca aaggagaaga actcttcact ggagttgtcc caattcttgt tgaattagat 60
   ggtgatgtta acggccacaa gttctctgtc agtggagagg gtgaaggtga tgcaacatac 120
   ggaaaactta ccctgaagtt catctgcact actggcaaac tgcctgttcc atggccaaca 180
   ctagtcacta ctctgtgcta tggtgttcaa tgcttttcaa gatacccgga tcatatgaaa 240
   cggcatgact ttttcaagag tgccatgccc gaaggttatg tacaggaaag gaccatcttc 300
   ttcaaagatg acggcaacta caagacacgt gctgaagtca agtttgaagg tgataccctt 360
   gttaatagaa tcgagttaaa aggtattgac ttcaaggaag atggcaacat tctgggacac 420
   aaattggaat acaactataa ctcacacaat gtatacatca tggcagacaa acaaaagaat 480
   ggaatcaaag tgaacttcaa gacccgccac aacattgaag atggaagcgt tcaactagca 540
   gaccattatc aacaaaatac tccaattggc gatggccctg tccttttacc agacaaccat 600
   tacctgtcca cacaatctgc cctttcgaaa gatcccaacg aaaagagaga ccacatggtc 660
   cttcttgagt ttgtaacagc tgctgggatt acacatggca tggatgaact gtacaac 717
<210> 9
   <211> 717
   <212> DNA
   <213> Artificial Sequence
<220>
   <223> Reduced number of UU and/or UA dinucleotides
<400> 9
   atggccagca agggcgagga actgttcacc ggcgtggtgc ccatcctggt ggagctggac 60
   ggcgacgtga acggccacaa gttcagcgtg agcggcgagg gcgaaggcga cgccacctac 120
   ggcaagctga ccctgaagtt catctgcacc accggcaagc tgcccgtgcc ctggcccacc 180
   ctggtgacca ccctgtgcta cggcgtgcag tgcttcagca gataccccga ccacatgaag 240
   cggcacgact tcttcaagag cgccatgccc gagggctacg tgcaggaacg gaccatcttc 300
   ttcaaggacg acggcaacta caagaccagg gccgaggtga agttcgaggg cgacacactg 360
   gtgaaccgga tcgagctgaa gggcatcgac ttcaaagagg acggcaacat cctgggccac 420
   aagctggaat acaactacaa cagccacaac gtgtacatca tggccgacaa gcagaagaac 480
   ggcatcaagg tcaacttcaa gacccggcac aacatcgagg acggcagcgt gcagctggcc 540
   gaccactacc agcagaacac ccccatcggc gacggccccg tgctgctgcc cgacaaccac 600
   tacctgagca cccagagcgc cctgagcaag gaccccaacg agaagcggga ccacatggtg 660
   ctgctggaat tcgtgacagc cgccggaatc acccacggca tggacgagct gtacaac 717
<210> 10
   <211> 684
   <212> DNA
   <213> Montastrea cavernosa
<400> 10
   atgggcgtga tcaagcccga catgaagatc aagctgcgga tggagggcgc cgtgaacggc      60
   cacaaattcg tgatcgaggg cgacgggaaa ggcaagccct ttgagggtaa gcagactatg     120
   gacctgaccg tgatcgaggg cgcccccctg cccttcgctt atgacattct caccaccgtg     180
   ttcgactacg gtaaccgtgt cttcgccaag taccccaagg acatccctga ctacttcaag     240
   cagaccttcc ccgagggcta ctcgtgggag cgaagcatga catacgagga ccagggaatc     300
   tgtatcgcta caaacgacat caccatgatg aagggtgtgg acgactgctt cgtgtacaaa     360
   atccgcttcg acggggtcaa cttccctgct aatggcccgg tgatgcagcg caagacccta     420
   aagtgggagc ccagtaccga gaagatgtac gtgcgggacg gcgtactgaa gggcgatgtt     480
   aatatggcac tgctcttgga gggaggcggc cactaccgct gcgacttcaa gaccacctac     540
   aaagccaaga aggtggtgca gcttcccgac taccacttcg tggaccaccg catcgagatc     600
   gtgagccacg acaaggacta caacaaagtc aagctgtacg agcacgccga agcccacagc     660
   ggactacccc gccaggccgg ctaa                                            684
<210> 11
   <211> 809
   <212> DNA
   <213> Artificial Sequence
<220>
   <223> Reduced number of UU and/or UA dinucleotides
<400> 11
   atgggcgtga tcaagcccga catgaagatc aagctgcgga tggagggcgc cgtgaacggc      60
   cacaaattcg tgatcgaggg cgacgggaaa ggcaagccct tcgagggcaa gcagacgatg     120
   gacctgaccg tgatcgaggg cgcccccctg cccttcgcct acgacatcct gaccaccgtg     180
   ttcgactacg gcaaccgtgt cttcgccaag taccccaagg acatccctga ctacttcaag     240
   cagaccttcc ccgagggcta ctcgtgggag cgaagcatga catacgagga ccagggaatc     300
   tgcatcgcga caaacgacat caccatgatg aagggtgtgg acgactgctt cgtgtacaaa     360
   atccgcttcg acggggtcaa cttccctgcc aatggcccgg tgatgcagcg caagaccctg     420
   aagtgggagc ccagcaccga gaagatgtac gtgcgggacg gcgtcctgaa gggcgatgtg     480
   aacatggcac tgctcctgga gggaggcggc cactaccgct gcgacttcaa gaccacctac     540
   aaagccaaga aggtggtgca gctgcccgac taccacttcg tggaccaccg catcgagatc     600
   gtgagccacg acaaggacta caacaaagtc aagctgtacg agcacgccga agcccacagc     660
   ggactgcccc gccaggccgg ctgaagtctc acggcttccc acccgaggtc gaggagcagg     720
   atgatggcac actgcccatg agctgtgctc aggagtctgg catggacaga caccccgctg     780
   cctgtgccag tgccaggatc aatgtgtga                                       809
<210> 12
   <211> 711
   <212> DNA
   <213> Clavularia species
<400> 12
   atggtgagca agggcgagga gaccacaatg ggcgtaatca agcccgacat gaagatcaag      60
   ctgaagatgg agggcaacgt gaatggccac gccttcgtga tcgagggcga gggcgagggc     120
   aagccctacg acggcaccaa caccatcaac ctggaggtga aggagggagc ccccctgccc     180
   ttctcctacg acattctgac caccgcgttc agttacggca acagggcctt caccaagtac     240
   cccgacgaca tccccaacta cttcaagcag tccttccccg agggctactc ttgggagcgc     300
   accatgacct tcgaggacaa gggcatcgtg aaggtgaagt ccgacatctc catggaggag     360
   gactccttca tctacgagat acacctcaag ggcgagaact tcccccccaa cggccccgtg     420
   atgcagaagg agaccaccgg ctgggacgcc tccaccgaga ggatgtacgt gcgcgacggc     480
   gtgctgaagg gcgacgtcaa gatgaagctg ctgctggagg gcggcggcca ccaccgcgtt     540
   gacttcaaga ccatctacag ggccaagaag gcggtgaagc tgcccgacta tcactttgtg     600
   gaccaccgca tcgagatcct gaaccacgac aaggactaca acaaggtgac cgtttacgag     660
   atcgccgtgg cccgcaactc caccgacggc atggacgagc tgtacaagta a              711
<210> 13
   <211> 712
   <212> DNA
   <213> Artificial Sequence
<220>
   <223> Reduced number of UU and/or UA dinucleotides
<400> 13
   atggtgagca agggcgagga gaccacaatg ggcgtgatca agcccgacat gaagatcaag      60
   ctgaagatgg agggcaacgt gaatggccac gccttcgtga tcgagggcga gggcgagggc     120
   aagccctacg acggcaccaa caccatcaac ctggaggtga aggagggagc ccccctgccc     180
   ttctcctacg acatcctgac caccgcgttc agctacggca acagggcctt caccaagtac     240
   cccgacgaca tccccaacta cttcaagcag tccttccccg agggctacag ctgggagcgc     300
   accatgacct tcgaggacaa gggcatcgtg aaggtgaagt ccgacatctc catggaggag     360
   gactccttca tctacgagat ccacctcaag ggcgagaact tcccccccaa cggccccgtg     420
   atgcagaagg agaccaccgg ctgggacgcc tccaccgaga ggatgtacgt gcgcgacggc     480
   gtgctgaagg gcgacgtcaa gatgaagctg ctgctggagg gcggcggcca ccaccgcgtg     540
   gacttcaaga ccatctacag ggccaagaag gcggtgaagc tgcccgacta tcacttcgtg     600
   gaccaccgca tcgagatcct gaaccacgac aaggactaca acaaggtgac cgtgtacgag     660
   atcgccgtgg cccgcaactc caccgacggc atggacgagc tgtacaagct ga             712
<210> 14
   <211> 1368
   <212> DNA
   <213> Firefly
<400> 14
   atggaagacg ccaaaaacat aaagaaaggc ccggcgccat tctatccgct ggaagatgga      60
   accgctggag agcaactgca taaggctatg aagagatacg ccctggttcc tggaacaatt     120
   gcttttacag atgcacatat cgaggtggac atcacttacg ctgagtactt cgaaatgtcc     180
   gttcggttgg cagaagctat gaaacgatat gggctgaata caaatcacag aatcgtcgta     240
   tgcagtgaaa actctcttca attctttatg ccggtgttgg gcgcgttatt tatcggagtt     300
   gcagttgcgc ccgcgaacga catttataat gaacgtgaat tgctcaacag tatgggcatt     360
   tcgcagccta ccgtggtgtt cgtttccaaa aaggggttgc aaaaaatttt gaacgtgcaa     420
   aaaaagctcc caatcatcca aaaaattatt atcatggatt ctaaaacgga ttaccaggga     480
   tttcagtcga tgtacacgtt cgtcacatct catctacctc ccggttttaa tgaatacgat     540
   tttgtgccag agtccttcga tagggacaag acaattgcac tgatcatgaa ctcctctgga     600
   tctactggtc tgcctaaagg tgtcgctctg cctcatagaa ctgcctgcgt gagattctcg     660
   catgccagag atcctatttt tggcaatcaa atcattccgg atactgcgat tttaagtgtt     720
   gttccattcc atcacggttt tggaatgttt actacactcg gatatttgat atgtggattt     780
   cgagtcgtct taatgtatag atttgaagaa gagctgtttc tgaggagcct tcaggattac     840
   aagattcaaa gtgcgctgct ggtgccaacc ctattctcct tcttcgccaa aagcactctg     900
   attgacaaat acgatttatc taatttacac gaaattgctt ctggtggcgc tcccctctct     960
   aaggaagtcg gggaagcggt tgccaagagg ttccatctgc caggtatcag gcaaggatat    1020
   gggctcactg agactacatc agctattctg attacacccg agggggatga taaaccgggc    1080
   gcggtcggta aagttgttcc attttttgaa gcgaaggttg tggatctgga taccgggaaa    1140
   acgctgggcg ttaatcaaag aggcgaactg tgtgtgagag gtcctatgat tatgtccggt    1200
   tatgtaaaca atccggaagc gaccaacgcc ttgattgaca aggatggatg gctacattct    1260
   ggagacatag cttactggga cgaagacgaa cacttcttca tcgttgaccg cctgaagtct    1320
   ctgattaagt acaaaggcta tcaggtggct cccgctgaat tggaatcc                 1368
<210> 15
   <211> 1650
   <212> DNA
   <213> Artificial Sequence
<220>
   <223> Reduced number of UU and/or UA dinucleotides
<400> 15
   atggaagacg ccaaaaacat caagaaaggc ccggcgccat tctacccgct ggaagatgga      60
   accgctggag agcaactgca caaggccatg aagagatacg ccctggtgcc tggaacaatc     120
   gcgttcacag atgcacacat cgaggtggac atcacctacg ctgagtactt cgaaatgtcc     180
   gtccggctgg cagaagccat gaaacgatac gggctgaaca caaatcacag aatcgtcgtg     240
   tgcagtgaaa actctctgca attcttcatg ccggtgctgg gcgcgctgtt catcggagtg     300
   gcagtcgcgc ccgcgaacga catctacaat gaacgtgaac tcctcaacag catgggcatc     360
   tcgcagccca ccgtggtgtt cgtgtccaaa aaggggctgc aaaaaatcct gaacgtgcaa     420
   aaaaagctcc caatcatcca aaaaatcatc atcatggaca gcaaaacgga ctaccaggga     480
   ttccagtcga tgtacacgtt cgtcacatct catctgcctc ccggcttcaa tgaatacgac     540
   ttcgtgccag agtccttcga cagggacaag acaatcgcac tgatcatgaa ctcctctgga     600
   agcactggtc tgcccaaagg tgtcgctctg cctcacagaa ctgcctgcgt gagattctcg     660
   catgccagag atcccatctt cggcaatcaa atcatcccgg acactgcgat cctgagtgtg     720
   gtcccattcc atcacggctt cggaatgttc acgacactcg gatacctgat ctgtggattc     780
   cgagtcgtcc tgatgtacag attcgaagaa gagctgttcc tgaggagcct ccaggactac     840
   aagatccaaa gtgcgctgct ggtgccaacc ctgttctcct tcttcgccaa aagcactctg     900
   atcgacaaat acgatctcag caatctgcac gaaatcgcct ctggtggcgc tcccctctcc     960
   aaggaagtcg gggaagcggt cgccaagagg ttccatctgc cagggatcag gcaaggatac    1020
   gggctcactg agacgacatc agccatcctg atcacacccg agggggatga caaaccgggc    1080
   gcggtcggga aagtggtccc attcttcgaa gcgaaggttg tggatctgga caccgggaaa    1140
   acgctgggcg ttaatcaaag aggcgaactg tgtgtgagag gtcccatgat catgtccggc    1200
   tacgtgaaca atccggaagc gaccaacgcc ctgatcgaca aggatggatg gctccactct    1260
   ggagacatcg cgtactggga cgaagacgaa cacttcttca tcgtggaccg cctgaagtct    1320
   ctgatcaagt acaaaggcta ccaggtggct cccgctgaac tcgaatccat cctgctccaa    1380
   caccccaaca tcttcgacgc aggtgtcgca ggtctgcccg acgatgacgc cggtgaactg    1440
   cccgccgccg tcgtggttct ggagcacgga aagacgatga cggaaaaaga gatcgtggac    1500
   tacgtcgcca gtcaagtaac aaccgcgaaa aagctgcgcg gaggagttgt gttcgtggac    1560
   gaagtgccga aaggtctgac cggaaaactc gacgcaagaa aaatcagaga gatcctcatc    1620
   aaggccaaga agggcggaaa gatcgccgtg                                    1650
<210> 16
   <211> 1656
   <212> DNA
   <213> Firefly
<400> 16
   atggaagatg ccaaaaacat taagaagggc ccagcgccat tctacccact cgaagacggg      60
   accgccggcg agcagctgca caaagccatg aagcgctacg ccctggtgcc cggcaccatc     120
   gcctttaccg acgcacatat cgaggtggac attacctacg ccgagtactt cgagatgagc     180
   gttcggctgg cagaagctat gaagcgctat gggctgaata caaaccatcg gatcgtggtg     240
   tgcagcgaga atagcttgca gttcttcatg cccgtgttgg gtgccctgtt catcggtgtg     300
   gctgtggccc cagctaacga catctacaac gagcgcgagc tgctgaacag catgggcatc     360
   agccagccca ccgtcgtatt cgtgagcaag aaagggctgc aaaagatcct caacgtgcaa     420
   aagaagctac cgatcataca aaagatcatc atcatggata gcaagaccga ctaccagggc     480
   ttccaaagca tgtacacctt cgtgacttcc catttgccac ccggcttcaa cgagtacgac     540
   ttcgtgcccg agagcttcga ccgggacaaa accatcgccc tgatcatgaa cagtagtggc     600
   agtaccggat tgcccaaggg cgtagcccta ccgcaccgca ccgcttgtgt ccgattcagt     660
   catgcccgcg accccatctt cggcaaccag atcatccccg acaccgctat cctcagcgtg     720
   gtgccatttc accacggctt cggcatgttc accacgctgg gctacttgat ctgcggcttt     780
   cgggtcgtgc tcatgtaccg cttcgaggag gagctattct tgcgcagctt gcaagactat     840
   aagattcaat ctgccctgct ggtgcccaca ctatttagct tcttcgctaa gagcactctc     900
   atcgacaagt acgacctaag caacttgcac gagatcgcca gcggcggggc gccgctcagc     960
   aaggaggtag gtgaggccgt ggccaaacgc ttccacctac caggcatccg ccagggctac    1020
   ggcctgacag aaacaaccag cgccattctg atcacccccg aaggggacga caagcctggc    1080
   gcagtaggca aggtggtgcc cttcttcgag gctaaggtgg tggacttgga caccggtaag    1140
   acactgggtg tgaaccagcg cggcgagctg tgcgtccgtg gccccatgat catgagcggc    1200
   tacgttaaca accccgaggc tacaaacgct ctcatcgaca aggacggctg gctgcacagc    1260
   ggcgacatcg cctactggga cgaggacgag cacttcttca tcgtggaccg gctgaagagc    1320
   ctgatcaaat acaagggcta ccaggtagcc ccagccgaac tggagagcat cctgctgcaa    1380
   caccccaaca tcttcgacgc cggggtcgcc ggcctgcccg acgacgatgc cggcgagctg    1440
   cccgccgcag tcgtcgtgct ggaacacggt aaaaccatga ccgagaagga gatcgtggac    1500
   tatgtggcca gccaggttac aaccgccaag aagctgcgcg gtggtgttgt gttcgtggac    1560
   gaggtgccta aaggactgac cggcaagttg gacgcccgca agatccgcga gattctcatt    1620
   aaggccaaga agggcggcaa gatcgccgtg taataa                              1656
<210> 17
   <211> 1653
   <212> DNA
   <213> Artificial Sequence
<220>
   <223> Reduced number of UU and/or UA dinucleotides
<400> 17
   atggaagatg ccaaaaacat caagaagggc ccagcgccat tctacccact cgaagacggg       60
   accgcaggcg agcagctgca caaagccatg aagcgctacg ccctggtgcc cggcaccatc      120
   gccttcaccg acgcacacat cgaggtggac atcacctacg ccgagtactt cgagatgagc      180
   gtgcggctgg cagaagccat gaagcgctac gggctgaaca caaaccatcg gatcgtggtg      240
   tgcagcgaga acagcctgca gttcttcatg cccgtgctgg gtgccctgtt catcggtgtg      300
   gctgtggccc cagccaacga catctacaac gagcgcgagc tgctgaacag catgggcatc      360
   agccagccca ccgtcgtgtt cgtgagcaag aaagggctgc aaaagatcct caacgtgcaa      420
   aagaagctgc cgatcatcca aaagatcatc atcatggaca gcaagaccga ctaccagggc      480
   ttccaaagca tgtacacctt cgtgacctcc cacctgccac ccggcttcaa cgagtacgac      540
   ttcgtgcccg agagcttcga ccgggacaaa accatcgccc tgatcatgaa cagcagtggc      600
   agcaccggac tgcccaaggg cgtggcactg ccgcaccgca ccgcctgtgt ccgattcagt      660
   catgcacgcg accccatctt cggcaaccag atcatccccg acaccgccat cctcagcgtg      720
   gtgccattcc accacggctt cggcatgttc accacgctgg gctactggat ctgcggcttc      780
   cgggtcgtgc tcatgtaccg cttcgaggag gagctgttcc tgcgcagcct gcaagactac      840
   aagatccaat ctgccctgct ggtgcccaca ctgttcagct tcttcgccaa gagcactctc      900
   atcgacaagt acgacctgag caacctgcac gagatcgcca gcggcggagc gccgctcagc      960
   aaggaggtgg gtgaggccgt ggccaaacgc ttccacctgc caggcatccg ccagggctac     1020
   ggcctgacag aaacaaccag cgccattctg atcacccccg aaggggacga caagcctggc     1080
   gcagtgggca aggtggtgcc cttcttcgag gccaaggtgg tggacctgga caccggcaag     1140
   acactgggtg tgaaccagcg cggcgagctg tgcgtccgtg gccccatgat catgagcggc     1200
   tacgtgaaca accccgaggc cacaaacgct ctcatcgaca aggacggctg gctgcacagc     1260
   ggcgacatcg cctactggga cgaggacgag cacttcttca tcgtggaccg gctgaagagc     1320
   ctgatcaaat acaagggcta ccaggtggcc ccagccgaac tggagagcat cctgctgcaa     1380
   caccccaaca tcttcgacgc cggagtcgcc ggactgccag acgacgatgc cggcgagctg     1440
   cccgcagcag tcgtcgtgct ggaacacggc aaaaccatga ccgagaagga gatcgtggac     1500
   tacgtggcca gccaggtgac aaccgccaag aagctgcgcg gtggtgtggt gttcgtggac     1560
   gaggtgccca aaggactgac cggcaagctg gacgcccgca agatccgcga gatcctcatc     1620
   aaggccaaga agggcggcaa gatcgccgtg tga                                  1653
<210> 18
   <211> 697
   <212> DNA
   <213> Puntellina plumate
<400> 18
   atggagagcg acgagagcgg cctgcccgcc atggagatcg agtgccgcat caccggcacc 60
   ctgaacggcg tggagttcga gctggtgggc ggcggagagg gcacccccga gcagggccgc 120
   atgaccaaca agatgaagag caccaaaggc gccctgacct tcagccccta cctgctgagc 180
   cacgtgatgg gctacggctt ctaccacttc ggcacctacc ccagcggcta cgagaacccc 240
   ttcctgcacg ccatcaacaa cggcggctac accaacaccc gcatcgagaa gtacgaggac 300
   ggcggcgtgc tgcacgtgag cttcagctac cgctacgagg ccggccgcgt gatcggcgac 360
   ttcaaggtga tgggcaccgg cttccccgag gacagcgtga tcttcaccga caagatcatc 420
   cgcagcaacg ccaccgtgga gcacctgcac cccatgggcg ataacgatct ggatggcagc 480
   ttcacccgca ccttcagcct gcgcgacggc ggctactaca gctccgtggt ggacagccac 540
   atgcacttca agagcgccat ccaccccagc atcctgcaga acgggggccc catgttcgcc 600
   ttccgccgcg tggaggagga tcacagcaac accgagctgg gcatcgtgga gtaccagcac 660
   gccttcaaga ccccggatgc agatgccggt gaagaaa 697
<210> 19
   <211> 700
   <212> DNA
   <213> Artificial Sequence
<220>
   <223> Reduced number of UU and/or UA dinucleotides
<400> 19
   atggagagcg acgagagcgg cctgcccgcc atggagatcg agtgccgcat caccggcacc 60
   ctgaacggcg tggagttcga gctggtgggc ggcggagagg gcacccccga gcagggccgc 120
   atgaccaaca agatgaagag caccaaaggc gccctgacct tcagccccta cctgctgagc 180
   cacgtgatgg gctacggctt ctaccacttc ggcacctacc ccagcggcta cgagaacccc 240
   ttcctgcacg ccatcaacaa cggcggctac accaacaccc gcatcgagaa gtacgaggac 300
   ggcggcgtgc tgcacgtgag cttcagctac cgctacgagg ccggccgcgt gatcggcgac 360
   ttcaaggtga tgggcaccgg cttccccgag gacagcgtga tcttcaccga caagatcatc 420
   cgcagcaacg ccaccgtgga gcacctgcac cccatgggcg acaacgacct ggatggcagc 480
   ttcacccgca ccttcagcct gcgcgacggc ggctactaca gctccgtggt ggacagccac 540
   atgcacttca agagcgccat ccaccccagc atcctgcaga acgggggccc catgttcgcc 600
   ttccgccgcg tggaggagga tcacagcaac accgagctgg gcatcgtgga gtaccagcac 660
   gccttcaaga ccccggatgc agatgccggt gaagaactga 700
<210> 20
   <211> 698
   <212> DNA
   <213> Discosoma species
<400> 20
   atgagcgagc tgatcaagga gaacatgcac atgaagctgt acatggaggg caccgtgaac 60
   aaccaccact tcaagtgcac atccgagggc gaaggcaagc cctacgaggg cacccagacc 120
   atgaagatca aggtggtcga gggcggccct ctccccttcg ccttcgacat cctggctacc 180
   agcttcatgt acggcagcaa agccttcatc aaccacaccc agggcatccc cgacttcttt 240
   aagcagtcct tccctgaggg cttcacatgg gagagaatca ccacatacga agacgggggc 300
   gtgctgaccg ctacccagga caccagcttc cagaacggct gcatcatcta caacgtcaag 360
   atcaacgggg tgaacttccc atccaacggc cctgtgatgc agaagaaaac acgcggctgg 420
   gaggccaaca ccgagatgct gtaccccgct gacggcggcc tgagaggcca cagccagatg 480
   gccctgaagc tcgtgggcgg gggctacctg cactgctcct tcaagaccac atacagatcc 540
   aagaaacccg ctaagaacct caagatgccc ggcttccact tcgtggacca cagactggaa 600
   agaatcaagg aggccgacaa agagacctac gtcgagcagc acgagatggc tgtggccaag 660
   tactgcgacc tccctagcaa actggggcac agagatga 698
<210> 21
   <211> 694
   <212> DNA
   <213> Artificial Sequence
<220>
   <223> Reduced number of UU and/or UA dinucleotides
<400> 21
   atgagcgagc tgatcaagga gaacatgcac atgaagctgt acatggaggg caccgtgaac      60
   aaccaccact tcaagtgcac atccgagggc gaaggcaagc cctacgaggg cacccagacc     120
   atgaagatca aggtggtcga gggcggccca ctccccttcg ccttcgacat cctggccacc     180
   agcttcatgt acggcagcaa agccttcatc aaccacaccc agggcatccc cgacttcttc     240
   aagcagtcct tccctgaggg cttcacatgg gagagaatca ccacatacga agacgggggc     300
   gtgctgaccg ccacccagga caccagcttc cagaacggct gcatcatcta caacgtcaag     360
   atcaacgggg tgaacttccc atccaacggc cctgtgatgc agaagaaaac acgcggctgg     420
   gaggccaaca ccgagatgct gtaccccgct gacggcggcc tgagaggcca cagccagatg     480
   gccctgaagc tcgtgggcgg gggctacctg cactgctcct tcaagaccac atacagatcc     540
   aagaaacccg ccaagaacct caagatgccc ggcttccact tcgtggacca cagactggaa     600
   agaatcaagg aggccgacaa agagacctac gtcgagcagc acgagatggc tgtggccaag     660
   tactgcgacc tcccaagcaa actggggcac agac                                  694
<210> 22
   <211> 681
   <212> DNA
   <213> Hepatitis B virus
<400> 22
   atggagaaca caacatcagg attcctagga cccctgctcg tgttacaggc ggggtttttc      60
   ttgttgacaa gaatcctcac aataccacag agtctagact cgtggtggac ttctctcaat     120
   tttctagggg gagcacccac gtgtcctggc ccaaattcgc agtccccaac ctccaatcac     180
   tcaccaacct cttgtcctcc aatttgtcct ggctatcgct ggatgtgtct gcggcgtttt     240
   atcatattcc tcttcatcct gctgctatgc ctcatcttct tgttggttct tctggactac     300
   caaggtatgt tgcccgtttg tcctctactt ccaggaacat caactaccag cacgggacca     360
   tgcaagacct gcacgattcc tgctcaagga acctctatgt ttccctcctg ttgctgtaca     420
   aaaccttcgg acggaaactg cacttgtatt cccatcccat catcctgggc tttcgcaaga     480
   ttcctatggg agtgggcctc agtccgtttc tcctggctca gtttactagt gccatttgtt     540
   cagtggttcg tagggctttc ccccactgtt tggctttcag ttatatggat gatgtggtat     600
   tgggggccaa gtctgtacaa catcttgagt ccctttttac ctctattacc aattttcttt     660
   tgtctttggg tatacatttg a                                               681
<210> 23
   <211> 681
   <212> DNA
   <213> Artificial Sequence
<220>
   <223> Reduced number of UU and/or UA dinucleotides
<400> 23
   atggagaaca ccaccagcgg cttcctgggc cctctgctgg tgctgcaggc cggcttcttc    60
   ctgctgaccc gcatcctgac catcccccag agcctggaca gctggtggac cagcctgaac   120
   ttcctgggcg gagccccaac ctgtcccggc cccaacagcc agagccccac cagcaaccac   180
   agcccaacca gctgcccacc catctgtccc ggctaccggt ggatgtgcct gcggcggttc   240
   atcatcttcc tgttcatcct gctgctgtgc ctgatcttcc tcctggtgct cctggactac   300
   cagggcatgc tgcccgtgtg tcctctgctg cctggcacca gcaccacctc caccggcccc   360
   tgcaagacct gcacaatccc cgcccaggga accagcatgt tcccaagctg ctgctgcacc   420
   aagcccagcg acggcaactg cacctgcatc cccatcccaa gcagctgggc cttcgccaga   480
   ttcctgtggg agtgggcctc cgtgagattc agctggctgt cactgctggt gcccttcgtg   540
   cagtggttcg tgggcctgag cccaacagtg tggctgagcg tgatctggat gatgtggtac   600
   tggggaccca gcctgtacaa catcctgagc cccttcctgc ccctgctgcc catcttcttc   660
   tgcctgtggg tgtacatctg a                                             681
<210> 24
   <211> 681
   <212> DNA
   <213> Artificial Sequence
<220>
   <223> Reduced number of UU and/or UA dinucleotides
<400> 24
   atggagaaca caacatcagg attcctcgga cccctgctcg tgctgcaggc ggggttcttc 60
   ctgctcacaa gaatcctcac aatcccacag agtctggact cgtggtggac gtctctcaac 120
   ttcctcgggg gagcacccac gtgtcctggc ccaaactcgc agtccccaac ctccaatcac 180
   tcaccaacct cgtgtcctcc aatctgtcct ggctaccgct ggatgtgtct gcggcgcttc 240
   atcatcttcc tcttcatcct gctgctgtgc ctcatcttcc tgctcgtcct cctggactac 300
   caagggatgc tgcccgtctg tcctctgctg ccaggaacat caaccaccag cacgggacca 360
   tgcaagacct gcacgatccc tgctcaagga accagcatgt tcccctcctg ctgctgcaca 420
   aaaccatcgg acggaaactg cacctgcatc cccatcccat catcctgggc cttcgcaaga 480
   ttcctctggg agtgggcctc agtccggttc tcctggctca gtctcctggt gccattcgtg 540
   cagtggttcg tcgggctgtc ccccactgtg tggctgtcag tgatctggat gatgtggtac 600
   tgggggccaa gtctgtacaa catcctcagt cccttcctgc ctctgctgcc aatcttcttc 660
   tgtctgtggg tgtacatctg a 681
<210> 25
   <211> 567
   <212> DNA
   <213> Homo sapiens
<400> 25
   atggccttga cctttgcttt actggtggcc ctcctggtgc tcagctgcaa gtcaagctgc 60
   tctgtgggct gtgatctgcc tcaaacccac agcctgggta gcaggaggac cttgatgctc 120
   ctggcacaga tgaggagaat ctctcttttc tcctgcttga aggacagaca tgactttgga 180
   tttccccagg aggagtttgg caaccagttc caaaaggctg aaaccatccc tgtcctccat 240
   gagatgatcc agcagatctt caatctcttc agcacaaagg actcatctgc tgcttgggat 300
   gagaccctcc tagacaaatt ctacactgaa ctctaccagc agctgaatga cctggaagcc 360
   tgtgtgatac agggggtggg ggtgacagag actcccctga tgaaggagga ctccattctg 420
   gctgtgagga aatacttcca aagaatcact ctctatctga aagagaagaa atacagccct 480
   tgtgcctggg aggttgtcag agcagaaatc atgagatctt tttctttgtc aacaaacttg 540
   caagaaagtt taagaagtaa ggaatga 567
<210> 26
   <211> 567
   <212> DNA
   <213> Artificial Sequence
<220>
   <223> Reduced number of UU and/or UA dinucleotides
<400> 26
   atggccctga ccttcgccct gctggtggct ctgctggtgc tgagctgcaa gagcagctgc      60
   agcgtgggct gcgatctgcc tcagacccac agcctgggca gcagacggac actgatgctg     120
   ctggcccaga tgcggcggat cagcctgttc agctgcctga aggaccggca cgacttcggc     180
   ttcccccagg aagagttcgg caaccagttc cagaaggccg agacaatccc cgtgctgcac     240
   gagatgatcc agcagatctt caacctgttc agcaccaagg acagcagcgc cgcctgggac     300
   gagacactgc tggacaagtt ctacaccgag ctgtaccagc agctgaacga cctggaagcc     360
   tgcgtgatcc agggcgtggg cgtgaccgag acacccctga tgaaggaaga cagcatcctg     420
   gccgtgcgga agtacttcca gcggatcacc ctgtacctga aagagaagaa gtacagcccc     480
   tgcgcctggg aagtggtccg ggccgagatc atgcggagct tcagcctgag caccaacctg     540
   caggaaagcc tgcggagcaa agagtga                                         567
<210> 27
   <211> 612
   <212> DNA
   <213> Homo sapiens
<400> 27
   atggctggac ctgccaccca gagccccatg aagctgatgg ccctgcagct gctgctgtgg      60
   cacagtgcac tctggacagt gcaggaagcc acccccctgg gccctgccag ctccctgccc    120
   cagagcttcc tgctcaagtg cttagagcaa gtgaggaaga tccagggcga tggcgcagcg     180
   ctccaggaga agctgtgtgc cacctacaag ctgtgccacc ccgaggagct ggtgctgctc     240
   ggacactctc tgggcatccc ctgggctccc ctgagcagct gccccagcca ggccctgcag     300
   ctggcaggct gcttgagcca actccatagc ggccttttcc tctaccaggg gctcctgcag     360
   gccctggaag ggatctcccc cgagttgggt cccaccttgg acacactgca gctggacgtc     420
   gccgactttg ccaccaccat ctggcagcag atggaagaac tgggaatggc ccctgccctg     480
   cagcccaccc agggtgccat gccggccttc gcctctgctt tccagcgccg ggcaggaggg     540
   gtcctagttg cctcccatct gcagagcttc ctggaggtgt cgtaccgcgt tctacgccac     600
   cttgcccagc cc                                                         612
<210> 28
   <211> 612
   <212> DNA
   <213> Artificial Sequence
<220>
   <223> Reduced number of UU and/or UA dinucleotides
<400> 28
   atggctggac ctgccaccca gagccccatg aagctgatgg ccctgcagct gctgctgtgg 60
   cacagtgcac tctggacagt gcaggaagcc acccccctgg gccctgccag ctccctgccc 120
   cagagcttcc tgctcaagtg cctggagcaa gtgaggaaga tccagggcga tggcgcagcg 180
   ctccaggaga agctgtgtgc cacctacaag ctgtgccacc ccgaggagct ggtgctgctc 240
   ggacactctc tgggcatccc ctgggctccc ctgagcagct gccccagcca ggccctgcag 300
   ctggcaggct gcctgagcca actccacagc ggcctcttcc tctaccaggg gctcctgcag 360
   gccctggaag ggatctcccc cgagctgggt cccaccctgg acacactgca gctggacgtc 420
   gccgacttcg ccaccaccat ctggcagcag atggaagaac tgggaatggc ccctgccctg 480
   cagcccaccc agggtgccat gccggccttc gcctctgcct tccagcgccg ggcaggaggg 540
   gtcctggtgg cctcccatct gcagagcttc ctggaggtgt cgtaccgcgt gctccgccac 600
   ctcgcccagc cc 612
<210> 29
   <211> 123
   <212> DNA
   <213> Mus musculus
<400> 29
   cagagccatg gcttcccgcc ggaggtggag gagcaggatg atggcacgct gcccatgtct 60
   tgtgcccagg agagcgggat ggaccgtcac cctgcagcct gtgcttctgc taggatcaat 120
   gtg 123
<210> 30
   <211> 125
   <212> DNA
   <213> Artificial Sequence
<220>
   <223> Reduced number of UU and/or UA dinucleotides
<400> 30
   agtctcacgg cttcccaccc gaggtcgagg agcaggatga tggcacactg cccatgagct 60
   gtgctcagga gtctggcatg gacagacacc ccgctgcctg tgccagtgcc aggatcaatg 120
   tgtga 125
<210> 31
   <211> 806
   <212> DNA
   <213> Artificial Sequence
<220>
   <223> MODC destabilised and UU/UA reduced sequence of Montastrea cavernosa GFP (green fluorescent protein)
<400> 31
   atgggcgtga tcaagcccga catgaagatc aagctgcgga tggagggcgc cgtgaacggc      60
   cacaaattcg tgatcgaggg cgacgggaaa ggcaagccct tcgagggcaa gcagacgatg     120
   gacctgaccg tgatcgaggg cgcccccctg cccttcgcct acgacatcct gaccaccgtg     180
   ttcgactacg gcaaccgtgt cttcgccaag taccccaagg acatccctga ctacttcaag     240
   cagaccttcc ccgagggcta ctcgtgggag cgaagcatga catacgagga ccagggaatc     300
   tgcatcgcga caaacgacat caccatgatg aagggtgtgg acgactgctt cgtgtacaaa     360
   atccgcttcg acggggtcaa cttccctgcc aatggcccgg tgatgcagcg caagaccctg     420
   aagtgggagc ccagcaccga gaagatgtac gtgcgggacg gcgtcctgaa gggcgatgtg     480
   aacatggcac tgctcctgga gggaggcggc cactaccgct gcgacttcaa gaccacctac     540
   aaagccaaga aggtggtgca gctgcccgac taccacttcg tggaccaccg catcgagatc     600
   gtgagccacg acaaggacta caacaaagtc aagctgtacg agcacgccga agcccacagc     660
   ggactgcccc gccaggccgg cagtctcacg gcttcccacc cgaggtcgag gagcaggatg     720
   atggcacact gcccatgagc tgtgctcagg agtctggcat ggacagacac cccgctgcct     780
   gtgccagtgc caggatcaat gtgtga                                          806
<210> 32
   <211> 834
   <212> DNA
   <213> Artificial Sequence
<220>
   <223> MODC destabilised and UU/UA reduced sequence of Clavularia species GFP
<400> 32
   atggtgagca agggcgagga gaccacaatg ggcgtgatca agcccgacat gaagatcaag      60
   ctgaagatgg agggcaacgt gaatggccac gccttcgtga tcgagggcga gggcgagggc     120
   aagccctacg acggcaccaa caccatcaac ctggaggtga aggagggagc ccccctgccc     180
   ttctcctacg acatcctgac caccgcgttc agctacggca acagggcctt caccaagtac     240
   cccgacgaca tccccaacta cttcaagcag tccttccccg agggctacag ctgggagcgc     300
   accatgacct tcgaggacaa gggcatcgtg aaggtgaagt ccgacatctc catggaggag     360
   gactccttca tctacgagat ccacctcaag ggcgagaact tcccccccaa cggccccgtg     420
   atgcagaagg agaccaccgg ctgggacgcc tccaccgaga ggatgtacgt gcgcgacggc     480
   gtgctgaagg gcgacgtcaa gatgaagctg ctgctggagg gcggcggcca ccaccgcgtg     540
   gacttcaaga ccatctacag ggccaagaag gcggtgaagc tgcccgacta tcacttcgtg     600
   gaccaccgca tcgagatcct gaaccacgac aaggactaca acaaggtgac cgtgtacgag     660
   atcgccgtgg cccgcaactc caccgacggc atggacgagc tgtacaagca gtctcacggc     720
   ttcccacccg aggtcgagga gcaggatgat ggcacactgc ccatgagctg tgctcaggag     780
   tctggcatgg acagacaccc cgctgcctgt gccagtgcca ggatcaatgt gtga           834
<210> 33
   <211> 1775
   <212> DNA
   <213> Artificial Sequence
<220>
   <223> MODC destabilised and UU/UA reduced sequence of firefly luciferase
<400> 33
   atggaagacg ccaaaaacat caagaaaggc ccggcgccat tctacccgct ggaagatgga 60
   accgctggag agcaactgca caaggccatg aagagatacg ccctggtgcc tggaacaatc 120
   gcgttcacag atgcacacat cgaggtggac atcacctacg ctgagtactt cgaaatgtcc 180
   gtccggctgg cagaagccat gaaacgatac gggctgaaca caaatcacag aatcgtcgtg 240
   tgcagtgaaa actctctgca attcttcatg ccggtgctgg gcgcgctgtt catcggagtg 300
   gcagtcgcgc ccgcgaacga catctacaat gaacgtgaac tcctcaacag catgggcatc 360
   tcgcagccca ccgtggtgtt cgtgtccaaa aaggggctgc aaaaaatcct gaacgtgcaa 420
   aaaaagctcc caatcatcca aaaaatcatc atcatggaca gcaaaacgga ctaccaggga 480
   ttccagtcga tgtacacgtt cgtcacatct catctgcctc ccggcttcaa tgaatacgac 540
   ttcgtgccag agtccttcga cagggacaag acaatcgcac tgatcatgaa ctcctctgga 600
   agcactggtc tgcccaaagg tgtcgctctg cctcacagaa ctgcctgcgt gagattctcg 660
   catgccagag atcccatctt cggcaatcaa atcatcccgg acactgcgat cctgagtgtg 720
   gtcccattcc atcacggctt cggaatgttc acgacactcg gatacctgat ctgtggattc 780
   cgagtcgtcc tgatgtacag attcgaagaa gagctgttcc tgaggagcct ccaggactac 840
   aagatccaaa gtgcgctgct ggtgccaacc ctgttctcct tcttcgccaa aagcactctg 900
   atcgacaaat acgatctcag caatctgcac gaaatcgcct ctggtggcgc tcccctctcc 960
   aaggaagtcg gggaagcggt cgccaagagg ttccatctgc cagggatcag gcaaggatac 1020
   gggctcactg agacgacatc agccatcctg atcacacccg agggggatga caaaccgggc 1080
   gcggtcggga aagtggtccc attcttcgaa gcgaaggttg tggatctgga caccgggaaa 1140
   acgctgggcg ttaatcaaag aggcgaactg tgtgtgagag gtcccatgat catgtccggc 1200
   tacgtgaaca atccggaagc gaccaacgcc ctgatcgaca aggatggatg gctccactct 1260
   ggagacatcg cgtactggga cgaagacgaa cacttcttca tcgtggaccg cctgaagtct 1320
   ctgatcaagt acaaaggcta ccaggtggct cccgctgaac tcgaatccat cctgctccaa 1380
   caccccaaca tcttcgacgc aggtgtcgca ggtctgcccg acgatgacgc cggtgaactg 1440
   cccgccgccg tcgtggttct ggagcacgga aagacgatga cggaaaaaga gatcgtggac 1500
   tacgtcgcca gtcaagtaac aaccgcgaaa aagctgcgcg gaggagttgt gttcgtggac 1560
   gaagtgccga aaggtctgac cggaaaactc gacgcaagaa aaatcagaga gatcctcatc 1620
   aaggccaaga agggcggaaa gatcgccgtg agtctcacgg cttcccaccc gaggtcgagg 1680
   agcaggatga tggcacactg cccatgagct gtgctcagga gtctggcatg gacagacacc 1740
   ccgctgcctg tgccagtgcc aggatcaatg tgtga 1775
<210> 34
   <211> 1775
   <212> DNA
   <213> Artificial Sequence
<220>
   <223> MODC destabilised and UU/UA reduced sequence of firefly luciferase
<400> 34
   atggaagatg ccaaaaacat caagaagggc ccagcgccat tctacccact cgaagacggg 60
   accgcaggcg agcagctgca caaagccatg aagcgctacg ccctggtgcc cggcaccatc 120
   gccttcaccg acgcacacat cgaggtggac atcacctacg ccgagtactt cgagatgagc 180
   gtgcggctgg cagaagccat gaagcgctac gggctgaaca caaaccatcg gatcgtggtg 240
   tgcagcgaga acagcctgca gttcttcatg cccgtgctgg gtgccctgtt catcggtgtg 300
   gctgtggccc cagccaacga catctacaac gagcgcgagc tgctgaacag catgggcatc 360
   agccagccca ccgtcgtgtt cgtgagcaag aaagggctgc aaaagatcct caacgtgcaa 420
   aagaagctgc cgatcatcca aaagatcatc atcatggaca gcaagaccga ctaccagggc 480
   ttccaaagca tgtacacctt cgtgacctcc cacctgccac ccggcttcaa cgagtacgac 540
   ttcgtgcccg agagcttcga ccgggacaaa accatcgccc tgatcatgaa cagcagtggc 600
   agcaccggac tgcccaaggg cgtggcactg ccgcaccgca ccgcctgtgt ccgattcagt 660
   catgcacgcg accccatctt cggcaaccag atcatccccg acaccgccat cctcagcgtg 720
   gtgccattcc accacggctt cggcatgttc accacgctgg gctactggat ctgcggcttc 780
   cgggtcgtgc tcatgtaccg cttcgaggag gagctgttcc tgcgcagcct gcaagactac 840
   aagatccaat ctgccctgct ggtgcccaca ctgttcagct tcttcgccaa gagcactctc 900
   atcgacaagt acgacctgag caacctgcac gagatcgcca gcggcggagc gccgctcagc 960
   aaggaggtgg gtgaggccgt ggccaaacgc ttccacctgc caggcatccg ccagggctac 1020
   ggcctgacag aaacaaccag cgccattctg atcacccccg aaggggacga caagcctggc 1080
   gcagtgggca aggtggtgcc cttcttcgag gccaaggtgg tggacctgga caccggcaag 1140
   acactgggtg tgaaccagcg cggcgagctg tgcgtccgtg gccccatgat catgagcggc 1200
   tacgtgaaca accccgaggc cacaaacgct ctcatcgaca aggacggctg gctgcacagc 1260
   ggcgacatcg cctactggga cgaggacgag cacttcttca tcgtggaccg gctgaagagc 1320
   ctgatcaaat acaagggcta ccaggtggcc ccagccgaac tggagagcat cctgctgcaa 1380
   caccccaaca tcttcgacgc cggagtcgcc ggactgccag acgacgatgc cggcgagctg 1440
   cccgcagcag tcgtcgtgct ggaacacggc aaaaccatga ccgagaagga gatcgtggac 1500
   tacgtggcca gccaggtgac aaccgccaag aagctgcgcg gtggtgtggt gttcgtggac 1560
   gaggtgccca aaggactgac cggcaagctg gacgcccgca agatccgcga gatcctcatc 1620
   aaggccaaga agggcggcaa gatcgccgtg agtctcacgg cttcccaccc gaggtcgagg 1680
   agcaggatga tggcacactg cccatgagct gtgctcagga gtctggcatg gacagacacc 1740
   ccgctgcctg tgccagtgcc aggatcaatg tgtga 1775
<210> 35
   <211> 822
   <212> DNA
   <213> Artificial Sequence
<220>
   <223> MODC destabilised and UU/UA reduced sequence of Puntellina plumate GFP
<400> 35
   atggagagcg acgagagcgg cctgcccgcc atggagatcg agtgccgcat caccggcacc      60
   ctgaacggcg tggagttcga gctggtgggc ggcggagagg gcacccccga gcagggccgc      120
   atgaccaaca agatgaagag caccaaaggc gccctgacct tcagccccta cctgctgagc      180
   cacgtgatgg gctacggctt ctaccacttc ggcacctacc ccagcggcta cgagaacccc      240
   ttcctgcacg ccatcaacaa cggcggctac accaacaccc gcatcgagaa gtacgaggac      300
   ggcggcgtgc tgcacgtgag cttcagctac cgctacgagg ccggccgcgt gatcggcgac      360
   ttcaaggtga tgggcaccgg cttccccgag gacagcgtga tcttcaccga caagatcatc      420
   cgcagcaacg ccaccgtgga gcacctgcac cccatgggcg acaacgacct ggatggcagc      480
   ttcacccgca ccttcagcct gcgcgacggc ggctactaca gctccgtggt ggacagccac      540
   atgcacttca agagcgccat ccaccccagc atcctgcaga acgggggccc catgttcgcc      600
   ttccgccgcg tggaggagga tcacagcaac accgagctgg gcatcgtgga gtaccagcac      660
   gccttcaaga ccccggatgc agatgccggt gaagaacagt ctcacggctt cccacccgag      720
   gtcgaggagc aggatgatgg cacactgccc atgagctgtg ctcaggagtc tggcatggac      780
   agacaccccg ctgcctgtgc cagtgccagg atcaatgtgt ga                         822
<210> 36
   <211> 819
   <212> DNA
   <213> Artificial Sequence
<220>
   <223> MODC destabilised and UU/UA reduced sequence of Discosoma RFP (red fluorescent protein)
<400> 36
   atgagcgagc tgatcaagga gaacatgcac atgaagctgt acatggaggg caccgtgaac        60
   aaccaccact tcaagtgcac atccgagggc gaaggcaagc cctacgaggg cacccagacc       120
   atgaagatca aggtggtcga gggcggccca ctccccttcg ccttcgacat cctggccacc       180
   agcttcatgt acggcagcaa agccttcatc aaccacaccc agggcatccc cgacttcttc       240
   aagcagtcct tccctgaggg cttcacatgg gagagaatca ccacatacga agacgggggc       300
   gtgctgaccg ccacccagga caccagcttc cagaacggct gcatcatcta caacgtcaag       360
   atcaacgggg tgaacttccc atccaacggc cctgtgatgc agaagaaaac acgcggctgg       420
   gaggccaaca ccgagatgct gtaccccgct gacggcggcc tgagaggcca cagccagatg       480
   gccctgaagc tcgtgggcgg gggctacctg cactgctcct tcaagaccac atacagatcc       540
   aagaaacccg ccaagaacct caagatgccc ggcttccact tcgtggacca cagactggaa       600
   agaatcaagg aggccgacaa agagacctac gtcgagcagc acgagatggc tgtggccaag       660
   tactgcgacc tcccaagcaa actggggcac agacagtctc acggcttccc acccgaggtc       720
   gaggagcagg atgatggcac actgcccatg agctgtgctc aggagtctgg catggacaga       780
   caccccgctg cctgtgccag tgccaggatc aatgtgtga                              819